# FASCICULE DE BREVET EUROPEEN

(11) **EP 0 994 192 B1**
(45) Date de publication et mention de la délivrance du brevet: **23.04.2003**
(21) Numéro de dépôt: 99402456.0
(22) Date de dépôt: 07.10.1999
(51) Int. Cl.: C12N 15/81, C12N 1/19

(54) **Cassette de transformation de la levure**
Transformationskassetten für Hefe
Transformation cassette for yeast

(30) Priorité: 09.10.1998 FR 9812702
(43) Date de publication de la demande: 19.04.2000
(73) Titulaire: La Société Lesaffre et Cie, 75001 Paris (FR)
(72) Inventeur: Bauer, Jürgen, 59239 Thumeries (FR); Nacken, Valérie, 59700 Marq-en-Baroeul (FR); Loiez, Annie, 59000 Lille (FR)
(74) Mandataire: Warcoin, Jacques

(56) Documents cités:
- FR-A- 2 615 527
- SMITH J. ET AL.: "PCR-BASED GENE DISRUPTION IN SACCHAROMYCES - CEREVISIAE" METHODS IN MOLECULAR AND CELLULAR BIOLOGY, vol. 5, no. 5, 1995, pages 270-277, XP002108450
- ALANI E. ET AL.: "A method for gene disruption that allows repeated use of URA3 selection in the construction of multiply disrupted yeast strains." GENETICS, vol. 116, 1987, pages 541-545, XP002108451
- WACH A. ET AL.: "New heterologous modules for classical or PCR-based gene disruptions in Saccharomyces cerevisiae" YEAST, vol. 10, no. 13, 1994, pages 1790-1808, XP002108452
- ZHANG Z. ET AL.: "Plasmid stability in recombinant Saccharomyces cerevisiae" BIOTECHNOLOGY ADVANCES,GB,ELSEVIER PUBLISHING, BARKING, vol. 14, no. 4, 1 janvier 1996 (1996-01-01), pages 401-435, XP004072548 ISSN: 0734-9750

## Description

La présente invention a pour objet une famille d'outils de transformation de la levure ne laissant dans la levure aucun fragment d'ADN exogène autre que des fragments codant pour des protéines d'intérêt.

La présente invention a pour objet une cassette d'intégration-excision permettant d'inactiver un ou des allèles d'un gène et/ou d'insérer un nouveau gène en laissant dans la souche hôte seulement de l'ADN de levure et éventuellement le nouveau gène.

La présente invention a aussi pour objet un plasmide replicatif multicopie dans la levure ne comportant pas de fragment d'ADN exogène inutile pour la fonction recherchée pour la levure.

L'invention concerne également un procédé de transformation de levures utilisant lesdits outils ainsi que les souches transformées obtenues.

L'amélioration de la productivité et de la robustesse des souches de levure, est un souci constant auquel les technologies de recombinaison d'ADN peuvent contribuer à apporter des réponses.

Le remplacement' de gène est une technique de biologie moléculaire fréquemment utilisée chez la levure. Un fragment d'ADN est cloné dans un vecteur puis il est introduit dans la cellule à transformer. Le fragment d'ADN s'intègre par recombinaison homologue dans un région ciblée du génome receveur (Orr-Weaver T., Szostak J. et Rothstein R., 1981, Proc. Natl. Acad. Sci. USA, 78, pp. 6354-6358). Cependant dans la pratique, l'événement de recombinaison est rare, et ne se produit que dans quelques cellules. C'est pourquoi des marqueurs de sélection sont insérés entre les séquences assurant la recombinaison afin de permettre, après transformation, d'isoler les cellules où l'intégration du fragment d'ADN s'est produite par mise en évidence des marqueurs correspondant, par exemple, à une résistance à un antibiotique. Mais ces marqueurs de sélection sont difficiles à éliminer, ce qui présente l'inconvénient, d'une part de ne pas pouvoir utiliser à nouveau le même marqueur pour une autre transformation, et d'autre part de laisser dans la cellule hôte des fragments d'ADN exogènes.

Le problème se complique encore pour les souches industrielles de *Saccharomyces cerevisiae* qui se distinguent des souches de laboratoire par le fait qu'elles sont à la fois aneuploïdes et polyploïdes, c'est-à-dire que de nombreux gènes sont présents en plusieurs exemplaires : copies multiples en tandem, copies multiples dispersées dans le génome, familles de gènes peu différents dans leur séquence et dont aucune différence d'activité n'a été mise en évidence comme le gène *SUC* par exemple (Olson M., 1991, Genome Structure and Organization, in *Saccharomyces cerevisiae* in the Molecular Biology of the Yeast Saccharomyces - Genome Dynamics, Protein Synthesis and Energetics ; Ed. Broach, Jones, Pringle, CSHL Press, New York).

Cette ploïdie particulière rend difficile toute manipulation visant à inactiver un gène par disruption ou délétion de ses allèles car il est nécessaire d'inactiver toutes les copies du gène, le plus souvent en répétant l'opération d'inactivation.

Le caractère prototrophe des souches industrielles ne permet pas d'utiliser des marqueurs dits "d'auxotrophie" et implique uniquement l'utilisation de marqueurs dominants dans toutes les transformations, mais dans ce cas, il faut soit disposer de plusieurs marqueurs différents, soit éliminer le marqueur après chaque transformation pour pouvoir le réutiliser pour une nouvelle transformation.

Un autre problème réside dans le fait que pour réaliser une transformation quelle qu'elle soit, la construction d'ADN utilisée comporte souvent de l'ADN non originaire de levure qui n'est pas toujours éliminé ou pas en totalité. De l'ADN étranger reste donc présent dans le génome de la levure. Or pour la commercialisation dans le domaine de l'industrie agro-alimentaire, il est important que les levures ne contiennent pas d'ADN non originaire de souches appartenant au même genre, de préférence à la même espèce de levure, à la seule exception éventuellement de la partie d'un gène d'intérêt codant pour une protéine non produite naturellement par la souche de levure, comme par exemple une xylanase, une malate perméase, une enzyme malolactique, une lactate deshydrogenase.

Chez la levure, l'intégration d'un gène d'intérêt dans un fragment d'ADN ou gène cible, se fait selon le principe de la recombinaison homologue. Pour cela une cassette d'intégration comporte un module comprenant au moins un gène marqueur de la levure et le gène à intégrer, ce module étant flanqué de part et d'autre de fragments d'ADN homologue à ceux des extrémités du site d'intégration ciblé. Ces fragments seront dits ci-après "recombinogéniques" car ils assureront la double recombinaison homologue permettant l'insertion de la cassette. Ces fragments recombinogéniques seront appelés ci-après SR (= Sequence Recombinogénique). Après transformation de la levure avec la cassette par des procédés appropriés, une recombinaison homologue entre les séquences recombinogéniques de la construction et les régions correspondantes du gène cible a pour résultat l'inactivation du gène cible causée par l'intégration simultanée de la construction, c'est-à-dire le remplacement du gène cible par la cassette d'intégration.

Cette technique s'applique également à l'inactivation d'un gène, qui peut être une disruption/interruption ou une délétion; dans le cas d'une délétion totale, l'ensemble du gène cible est échangé, dans le cas d'une disruption/interruption la séquence du gène cible est interrompue, et en général, elle s'accompagne d'une délétion plus ou moins importante ; c'est pourquoi on utilise parfois la terminologie "délétion totale ou partielle" pour désigner aussi bien la délétion que la disruption d'un gène, ayant pour résultat de bloquer ou de modifier son expression. Dans ces deux cas de figure, la cassette à insérer dans le gène cible peut ne comporter que le marqueur de sélection flanqué des fragments homologues recombinogéniques.

Le problème se pose alors d'éliminer les fragments exogènes inutiles, notamment les marqueurs ainsi introduits, dont la présence est généralement considérée comme inopportune. Pour cela, on peut utiliser le phénomène du "pop-out" ou excision spontanée chez la levure. C'est un événement de recombinaison intra-chromosomique entre des séquences identiques ou similaires qui peut se produire naturellement. Une boucle d'ADN se forme entre deux séquences similaires directes, puis est éjectée, laissant en place l'une des deux séquences recombinées. La fréquence de ce phénomène augmentant avec le degré d'identité des séquences, il est possible de favoriser l'élimination d'un fragment d'ADN, par exemple d'un marqueur de sélection, en le plaçant entre deux séquences identiques directes, dites séquences répétées directes (SRD). Ainsi, le marqueur est excisé alors qu'une séquence répétée directe est conservée.

Par exemple un article décrit une construction -moléculaire (pNKY51) permettant de disrupter ou de déléter un gène de levure. (E. Alani , L. Cao et N. Kleckner, "A method for gene disruption that allows repeated use of *URA3* selection in the construction of multiply disrupted yeast strains", Genetics, 116, pp. 541-545, août 1987). Le vecteur est un plasmide d'*Escherichia coli* contenant le gène *URA3* (marqueur) flanqué de séquences répétées directes issues de l'opéron histidine de la Salmonelle (*hisG*). Ce plasmide est digéré pour produire un module (*hisG-URA3-hisG*) qui est ensuite flanqué de chaque côté de séquences homologues recombinogéniques du gène cible à disrupter. La cassette ainsi réalisée est intégrée dans le gène cible d'une levure Ura⁻, et les souches ayant intégré la cassette peuvent être isolées par sélection des souches Ura⁺. Après excision de la cassette, les souches redeviennent Ura⁻.

La construction est utilisée pour disrupter ou déléter les gènes *TRP1*, *SPO13*, *HO*, *RAD50*, *LEU2* de la levure. Les transformations multiples sont réalisées soit par croisement de souches portant une transformation chacune, soit par une série de transformations par différentes constructions de disruption et par cycles répétés de sélection Ura⁺ et Ura⁻ dans une souche unique.

Une des deux séquences répétées directes de la Salmonelle *hisG* reste dans le génome après chaque transformation.

Cette méthode permet de réutiliser le marqueur (marqueur d'auxotrophie) pour une nouvelle transformation.

Ce même type de procédé est décrit dans le document EP 635 574 afin de transformer des bactéries, des levures ou des champignons la caractéristique essentielle du procédé étant, à la place du marqueur *URA3*, l'utilisation d'un autre gène marqueur spécifique, le gène *amdS*. Le marqueur en question semble intéressant dans le cas des souches de fungi, notamment *Aspergillus*, toutefois le système apparaît complexe d'utilisation chez les levures.

Le document EP 220009 décrit un procédé permettant l'intégration de cassettes de délétion totale ou partielle chez la levure en utilisant le système d'excision dit "Cre-lox", ce procédé mis au point chez l'animal est également utilisable dans les levures, les séquences équivalentes aux séquences répétées directes sont dans ce cas des séquences spécifiques dites "lox" et l'excision de la séquence d'ADN comprise entre les deux séquences "lox" s'effectue en présence d'une recombinase "Cre".

L'un des inconvénients de ce système est que l'une des séquences "lox" qui reste est un ADN exogène provenant d'un bactériophage.

Le document EP 814 165 décrit un système permettant, là encore, d'exciser un module contenant un marqueur par l'action d'un système enzymatique spécifique sur des séquences sensibles, le système étant très voisin du système "Cre-lox", dans la mesure où il utilise une recombinase spécifique à la SRD.

L'objet de la présente invention est de proposer des cassettes de transformation de la levure ne laissant aucun fragment exogène inutile d'ADN, et qui comportent d'une part au moins un marqueur de sélection qui est dominant et négatif; et d'autre part deux séquences répétées directes (SRD) non exogènes et non recombinogéniques avec le génome de la levure, ces 2 séquences répétées directes étant disposées de part et d'autre du fragment contenant le marqueur dominant négatif. Ce marqueur de contre-sélection dominant est de préférence un marqueur inductible. Ces cassettes d'ADN, objet de l'invention, comportent de préférence un second marqueur de sélection.

Ces cassettes d'ADN, objet de l'invention, comportent éventuellement au moins un gène d'intérêt avec, si besoin, les éléments nécessaires à son expression dans la levure (par exemple tout promoteur, enhanceur, et/ou terminateur appropriés). Tout ADN exogène (= hétérologue) pour la levure, autre que celui de ce gène d'intérêt, est considéré comme ADN exogène inutile.

Un objet de la présente invention est de proposer une cassette d'intégration/excision permettant l'intégration d'un gène d'intérêt et/ou l'inactivation d'un gène de l'hôte, utilisable dans les levures, qui résout les problèmes précédents, notamment qui permet d'obtenir des intégrations exemptes d'ADN exogène en ne laissant en place que de l'ADN de levure appartenant au même genre et éventuellement un gène d'intérêt. Dans la présente description, on entend désigner par "ADN exogène" un ADN qui provient d'un genre différent ou plus spécifiquement d'une espèce différente, du genre ou de l'espèce de la souche de levure transformée.

C'est pourquoi la présente invention concerne une cassette d'ADN destinée à l'intégration d'un gène d'intérêt ou à l'inactivation d'un gène dans les levures, caractérisée en ce qu'elle comporte :
- au moins un marqueur dominant négatif, de préférence deux marqueurs dominants de sélection : un marqueur positif et un marqueur négatif;
- deux séquences répétées directes (SRD) non exogènes et non recombinogéniques avec le génome de la souche hôte, ces deux séquences répétées directes flanquant le ou les marqueurs de sélection ;
- deux séquences d'ADN recombinogéniques (SR) correspondant au site d'insertion désiré dans la levure, flanquant en 5' et 3' les deux susdites séquences répétées directes ;
- éventuellement un (ou plusieurs) gène(s) d'intérêt, comportant, si besoin, les éléments nécessaires à son expression dans la cellule hôte, placé entre une séquence SRD et la séquence d'ADN recombinogénique (SR) adjacente.

Cette cassette d'intégration/excision permet la délétion totale ou partielle de gènes présents dans le génome de souches industrielles de levure et éventuellement l'intégration simultanée d'un nouveau gène sans qu'aucun fragment d'ADN étranger reste dans le génome de la souche ainsi transformée (hormis éventuellement le gène nouvellement cloné si c'est le cas). De préférence, ladite cassette d'intégration/excision sera conçue de manière à permettre de transformer plusieurs allèles, voire tous les allèles d'un gène l'un après l'autre sans qu'il y ait en principe de risque de voir les transformations se reproduire dans le même site. Il est vérifié systématiquement que la transformation de tous les allèles ou d'un nombre déterminé d'entre eux a bien eu lieu. La procédure de transformation peut être répétée plusieurs fois grâce à l'utilisation d'au moins une séquence recombinogénique (SR) différente à chaque fois, les deux séquences SR dirigeant l'intégration de la cassette. De préférence, on changera les deux séquences SR comme expliqué ci-après, si une seule est changée, ce sera de préférence la séquence SR choisie en 3' du site d'insertion.

Lorsque la cassette d'intégration/excision est destinée à l'intégration d'un gène d'intérêt, ceci implique, qu'après l'excision, demeurera le gène d'intérêt et une séquence SRD, cet ensemble étant flanqué des deux séquences recombinogéniques; lorsque la cassette est destinée à l'inactivation du gène, il peut s'agir de l'inactivation d'un gène par délétion totale ou partielle, dans ce cas, il ne reste qu'une séquence SRD flanquée des deux séquences recombinogéniques. Il est important, de manière générale, que la séquence SRD soit non codante, en effet située en 3' d'une séquence recombinogénique, elle pourrait être exprimée. Par séquence non codante, on entend une séquence qui n'est pas traduite sous forme d'un peptide.

Un problème souvent rencontré dans l'expression d'un ou plusieurs gènes d'intérêt à un niveau suffisant est le besoin d'un grand nombre de copies du ou des dits gènes d'intérêt avec les éléments permettant leur expression. Un autre objet de l'invention est de proposer un plasmide multicopie d'expression dans la levure, ne contenant pas d'ADN exogène inutile, pouvant être obtenu à l'aide d'un plasmide navette *E. coli*-levure comportant deux séquences répétées directes (SRD) non exogènes et non recombinogéniques avec le génome de la souche hôte.

Ce plasmide navette *E. coli*-levure, de préférence *E. coli-S. cerevisiae* est aussi un objet de l'invention, il est caractérisé en ce qu'il comporte :
- au moins un marqueur de sélection qui est dominant et négatif, de préférence deux marqueurs de sélection,
- deux séquences répétées directes SRD non exogènes et non recombinogéniques, ces deux séquences séparant dans le plasmide circulaire deux régions : d'une part la partie levure destinée à être conservée dans la levure hôte et à s'exprimer et d'autre part la partie ou région utilisée pour l'obtention du plasmide navette et destinée à être éliminée par excision.

De préférence, la région placée entre les deux séquences répétées directes et destinée à être éliminée par excision comporte :
- une origine de réplication de *E. coli*.
- un marqueur de sélection pour *E. coli*.
- un marqueur dominant négatif de préférence inductible, c'est-à-dire un gène codant pour un composé toxique pour la levure et exprimé dans des conditions particulières de milieu correspondant aux conditions d'induction.

La seconde région délimitée par les 2 séquences SRD et destinée à devenir le plasmide multicopie d'expression dans la levure comporte :
- une origine de réplication levure 2 micron,
- un marqueur de sélection, d'origine levure,
- un système d'expression d'au moins un gène d'intérêt.

Un objet de l'invention est aussi une cassette d'ADN sous forme d'un plasmide multicopie levure susceptible d'être obtenu après excision du plasmide navette *E. coli*-levure défini ci-dessus et comportant une séquence SRD non exogène et non recombinogénique avec le génome de la souche hôte, une origine de réplication levure 2 micron, un marqueur de sélection d'origine levure, un système d'expression d'au moins un gène d'intérêt.

Ces nouvelles cassettes selon l'invention comportent de préférence deux marqueurs de sélection, l'un deux étant alors un marqueur dominant négatif, de préférence inductible. Un marqueur dominant au sens de la présente invention est un marqueur de cellules prototrophes (ou non auxotrophes vis à vis de la protéine codée par le marqueur) permettant dans un milieu de croissance particulier adapté au marqueur, uniquement la croissance des clones ayant la modification recherchée. On parle aussi de sélection directe. On appelle marqueur dominant positif, un marqueur dont l'expression dans la cellule hôte assure la survie dans certaines conditions particulières, dans la plupart des cas il s'agira d'un marqueur de résistance, c'est à dire que lorsque le marqueur correspondant est exprimé, il permet à la cellule de résister, par exemple à des antibiotiques ou à d'autres substances toxiques. Par marqueur dominant négatif, on entend au contraire désigner en général, un marqueur dont l'expression est toxique pour les cellules hôtes dans des conditions particulières. Il peut s'agir notamment d'un marqueur rendu "inductible" codant pour un produit toxique, comme par exemple le gène *RTA* (Ricin Toxine A), placé sous le contrôle d'un promoteur inductible fortement réprimé par le glucose. Ce promoteur inductible peut être par exemple un promoteur d'un gène *GAL*, ou encore un promoteur hybride comme le promoteur *GAL10-CYC1*.

L'article de Frankel *et al*., "Selection and characterization of ricin toxin A-chain mutations in *Saccharomyces cerevisiae*", Molecular and Cellular Biology, fév. 1989, pp. 415-420, décrit une méthode de sélection de mutants produisant une toxine inactive après transformation par une construction d'ADN comportant le gène codant pour la chaîne polypeptidique A de la toxine du ricin sous contrôle du promoteur *GAL1* et induction de l'expression de ce gène.

L'utilisation d'un gène inductible codant pour un produit toxique comme le polypeptide de la chaîne A de la toxine du ricin selon la présente invention est différente, car ce gène est utilisé comme marqueur négatif permettant une contre sélection, c'est-à-dire une deuxième sélection des souches transformées par la cassette d'intégration ou le plasmide réplicatif objet de l'invention, mais ayant perdu le module d'excision. Bien entendu, il sera vérifié systématiquement que la survie des cellules de levure transformées en milieu inductible est due à la perte du module d'excision et non à une mutation du gène *RTA* ou du gène codant pour une autre protéine toxique, inactivant la toxine produite. Ceci peut être réalisé pour les souches transformées avec la cassette d'intégration d'une part par séquençage de la zone transformée et d'autre part par vérification de l'absence de fragments détectables du gène *RTA* au sein du génome de la souche transformée. Des vérifications similaires peuvent être conduites. concernant la perte de ses éléments de construction par le plasmide multicopie d'expression dans la levure.

Des exemples de marqueurs positifs et négatifs sont donnés ci-après de façon plus détaillée.

La présence de deux marqueurs dominants permet d'assurer une très grande efficacité au système constitué par la cassette/d'intégration, excision et elle permet de choisir des marqueurs universels pour les levures donnant une sélection directe aisée.

On aura compris que le marqueur positif permet de sélectionner directement les cellules transformées puis, après culture en milieu inducteur, le marqueur négatif permet de sélectionner directement les cellules dont le module d'excision aura été éliminé par "pop-out" ou excision.

L'excision du module comportant les deux marqueurs dans la cassette d'intégration/excision est effectuée grâce à la présence des séquences répétées directes (SRD) dont la structure constitue également l'un des éléments caractéristiques de la présente invention.

De la même manière, la présence de deux marqueurs permet une grande efficacité au système constitué par le plasmide navette *E. coli*-levure. On aura compris que le marqueur négatif inductible et dominant permet de sélectionner les cellules ne contenant qu'un ou des plasmides ayant excisé la partie utilisée pour la construction du plasmide navette grâce à la présence des séquences répétées directes (SRD), caractéristiques de la présente invention. Dans le cas de cette construction, l'excision élimine le marqueur dominant négatif, de préférence inductible. Le marqueur positif d'origine levure qui est dans l'autre région du plasmide navette délimitée par les 2 séquences SRD, est conservé et sert à maintenir une pression de sélection pour la conservation et l'expression des plasmides multicopies levure, obtenus après excision.

Ces séquences SRD seront, de préférence, choisies parmi :
- des séquences mosaïques de levure, de préférence appartenant au genre Saccharomyces et plus préférentiellement *Saccharomyces cerevisiae*, ou
- des séquences d'ADN de levure non présentes dans la souche hôte.

Par "séquence mosaïque" de levure, on entend désigner une séquence d'ADN constituée de fragments provenant de différentes séquences d'ADN d'une ou plusieurs souches de levure, appartenant à la même espèce, voire à la même sous-espèce.

Afin de s'assurer que les fragments en question permettent d'obtenir une " séquence mosaïque" non recombinogènique, chacun de ces fragments comportera de préférence, moins de 30 pb, et la séquence SRD comportera de 80 à 300 pb afin d'assurer l'excision. La longueur des fragments, à savoir de l'ordre de 10 à 30 pb, ne permet pas qu'ils jouent le rôle de fragments recombinogéniques, par contre la séquence globale SRD constituée de ces différents fragments permet une recombinaison avec une SRD identique, et donc l'excision du fragment d'ADN compris entre les-2 séquences SRD identiques. De préférence une séquence SRD comporte environ 90 à 200 pb ou 210 pb. Après excision, l'une des 2 séquences SRD demeure en place dans le génome, il est donc nécessaire que cette séquence soit constituée par de l'ADN de levure, c'est à dire par de l'ADN appartenant au même genre et de préférence à la même espèce de levure.

Comme cela a été indiqué précédemment, il est possible également d'utiliser comme séquences SRD des fragments d'un gène de levure qui est absent de la souche hôte industrielle de levure à transformer. Par exemple l'espèce *Saccharomyces cerevisiae* est décrite comme assimilant et fermentant le mélibiose de manière variable selon les souches. Toutes les souches industrielles de levure de boulangerie sont mélibiose "moins", par contre la plupart des souches industrielles de levure de brasserie sont mélibiose " plus ", alors que toutes ces souches appartiennent à l'espèce *Saccharomyces cerevisiae*. Le gène *MEL1* est en conséquence un bon candidat pour donner les séquences SRD d'une cassette de transformation d'une souche industrielle de levure de boulangerie, selon l'invention. Le gène *MEL1* est décrit dans le brevet EP 241 044, il s'agit d'un gène qui code pour l'enzyme α-galactosidase qui hydrolyse le mélibiose en galactose et glucose. Ce gène est absent des souches industrielles de levure de boulangerie qui, pour cette raison, ne sont pas capables de consommer la totalité du raffinose des mélasses. Dans ce cas, il est possible d'utiliser des fragments SRD plus longs, de l'ordre de 200 pb ou 210 pb par exemple, pour faciliter l'excision, mais en se rappelant que la séquence SRD restant après excision doit avoir été rendue non codante par tout moyen approprié tel que changement du cadre de lecture ou l'introduction de codons stop par exemple.

Comme déjà indiqué, dans la cassette d'intégration/excision les séquences SRD décrites précédemment sont flanquées, en 3' et 5', de séquences d'ADN recombinogéniques, c'est à dire permettant l'intégration dans le gène cible par double recombinaison homologue et qui sont appelées séquences SR. Ces deux fragments d'ADN sont préparés, bien entendu, à partir de séquences choisies dans le gène cible qui correspondront alors aux sites spécifiquement reconnus pour la recombinaison homologue. Le choix des séquences SR détermine ainsi le site d'insertion de la cassette.

Lorsqu'on veut intégrer de manière successive un même module d'excision avec éventuellement un gène d'intérêt dans différents allèles d'une même famille de gènes, il est nécessaire de choisir les séquences recombinogéniques de manière à ce qu'elles ne viennent pas se recombiner dans un allèle déjà transformé.

Pour ce faire, on réalisera des cassettes d'intégration/excision successives avec au moins une séquence recombinogénique qui n'est plus présente dans les sites précédents recombinés, de préférence la séquence recombinogénique non présente dans le site précédemment recombiné sera choisie en 3' de ce site. Par exemple on choisira des fragments en partant de l'extrémité 3'du gène cible pour la recombinaison puis en se rapprochant du centre dudit gène cible pour chaque cassette successive. De préférence, les 2 séquences SR seront changées à chaque fois selon cette technique appliquée en choisissant des fragments SR différents en partant des deux extrémités du site cible pour la recombinaison dans les différentes cassettes d'intégration successives.

Le marqueur d'intégration peut être tout marqueur dominant positif, c'est-à-dire qui permet aux levures le possédant de survivre à une pression de sélection. Ce sera de préférence un marqueur de résistance, c'est-à-dire un gène qui confère à la souche une résistance vis-à-vis d'un élément toxique. L'emploi d'un marqueur d'auxotrophie (gène nécessaire à la croissance sur milieu privé d'un élément nutritif) sera une solution préférée pour la maintenance et l'expression de plasmides multicopies levure, il nécessite que les levures industrielles concernées aient été rendues auxotrophes par des disruptions ou délétions ciblées, comme celles rendues possibles par la cassette d'intégration-excision selon la présente invention.

Parmi les marqueurs positifs dominants qui peuvent être utilisés à titre de marqueur de résistance à un composé toxique, on peut citer (le composé toxique étant cité en premier) :
- Cuivre : le gène *CUP1* de *Saccharomyces cerevisiae*, qui permet la résistance au cuivre et au cadmium. Son utilisation comme marqueur de sélection positif dominant a été montrée chez des souches sensibles au cuivre (Henderson *et al*. 1985, Curr. Genet, 9, pp. 133-138 ; Pentilä *et al*. 1987, Curr. Genet., 12, pp. 413-420 ; Hottiger *et al*. 1995, Yeast, 11, pp. 1-14).
- Cycloheximide : des allèles mutants du gène ribosomal *CYH2* de *Saccharomyces cerevisiae* permettent la résistance au cycloheximide lorsqu'ils sont présents avec le gène sauvage *CYH2* (l'homozygotie ne confère pas de résistance (Struhl *et al*. 1983, Gene. 26, pp. 231-242)) ;
- FK520 : le gène *MDR3,* originaire de la souris, codant pour la P-glycoprotéine, lorsqu'il est exprimé chez *Saccharomyces cerevisiae* lui confère une résistance au FK520 (un agent antifongique et immunosuppresseur) (Raymond *et al*. 1994, Mol. Cell. Biol. 14, pp. 277-286) ;
- Fluoroacétate : le gène *dehH1*, issu de *Moraxella sp*, exprimé chez *Saccharomyces cerevisiae* permet la résistance au fluoroacétate (van den Berg *et al*. 1997, Yeast, 13(6), pp. 551-559) ;
- Fluoro-phénylalanine: l'allèle dominant *ARO4-OFP*, qui est une mutation d'un nucléotide du gène *ARO4* de *Saccharomyces cerevisiae*, permet la résistance au mélange p-fluoro-DL-phénylalanine, ou o-fluoro-DL-phenylalanine, et tyrosine (la tyrosine permet de supprimer l'inhibition de l'expression de ce gène). Son efficacité comme marqueur de résistance à un analogue d'acide aminé a été montrée sur plusieurs souches de levures industrielles (Shimura *et al*. 1993, Enzyme Microb. Technol. 15, pp. 874-876) ;
- Formaldéhyde : le gène *SFA1* de *Saccharomyces cerevisiae*, lorsqu'il est surexprimé permet une résistance au formaldéhyde cinq à sept fois plus importante par rapport à la souche sauvage correspondante. Ce gène a été utilisé comme marqueur de sélection d'un plasmide multicopie (Wehner *et al*. 1993, Yeast, 9, pp. 783-785). Un des avantages de ce marqueur est le faible coût de la molécule toxique (van den Berg *et al*. 1997 - Yeast, 13(6), pp. 551-559) ;
- Généticine : le module KanMX, qui contient la séquence codante kan^{r} issue du transposon Tn903 d'*Escherichia coli* fusionnée aux séquences de contrôle transcriptionnelles du gène *TEF* du fungus filamenteux *Ashbya gossypii*, ou à tout autre promoteur et terminateur de transcription fonctionnels, confère à *Saccharomyces cerevisiae* la résistance à la généticine (G418) (Wach *et al*. 1994, Yeast, 10, pp. 1793-1808, parmi les nombreux utilisateurs de ce marqueur) ;
- Glyphosate : la partie codante du gène *aroA* de *Escherichia coli*, insérée entre des séquences promotrice et terminatrice de *Saccharomyces cerevisiae*, y permet la résistance au glyphosate (Kunze *et al*. 1989, Curr. Genet. 15, pp. 91-98) ;
- HygromycineB : le gène *hph,* originaire d'un plasmide d'*Escherichia coli*, placé sous le contrôle du promoteur du gène *CYC1* de *Saccharomyces cerevisiae* permet la résistance à l'hygromycine B (Gritz and Davies 1983, Gene. 25, pp. 179-188) ;
- Méthotrexate : le gène *Mdhfr,* codant pour une dihydrofolate reductase chez la souris, confère à *Saccharomyces cerevisiae* la résistance au méthotrexate (Zhu *et al*. 1986, Gene. 50, pp. 225-237) ;
- Phléomycine : le gène *Tn5ble*, issu de *Escherichia coli*, permet à *Saccharomyces cerevisiae* d'acquérir une résistance à la phléomycine. Ce gène est généralement utilisé avec le promoteur *TEF1* et le terminateur *CYC1* de *Saccharomyces cerevisiae* (Wenzel *et al*. 1992,Yeast, 8, pp. 667-668) ;
- Sulfométuron : le gène *SMR1-410*, allèle mutant du gène *ILV2* de *Saccharomyces cerevisiae*, permet la résistance à l'herbicide sulfométuron, de manière dominante chez des souches hétérozygotes diploïdes (Xiao *et al*. 1990, Plasmid. 23, pp. 67-70). Un nouvel allèle mutant a récemment été mis en évidence (Xie *et al*. 1996, FEMS Microbiol. Letters. 137, pp. 165-168).

Pour ce qui concerne le second marqueur, à savoir le marqueur négatif c'est-à-dire le marqueur de contre-sélection, il s'agira comme cela a été indiqué précédemment la plupart du temps d'un marqueur rendu "inductible" et codant en condition d'induction pour une molécule toxique pour la cellule de levure.

Un marqueur de contre-sélection avantageux est la partie codante du gène *RTA* (Ricin Toxine A), placée sous la dépendance d'un promoteur inductible à la fois fort et fortement réprimé par le glucose, comme par exemple le promoteur *GAL10-CYC1* et d'un terminateur *PGK1*. Le principe de ces constructions à partir de promoteur et terminateur de levure est d'utiliser un promoteur et un terminateur issus de gènes différents, de manière à minimiser les risques de recombinaison non désirée dans le génome de la levure.

Bien entendu, il est vérifié ensuite par séquençage de la région transformée l'absence d'ADN exogène, c'est-à-dire que l'excision a bien eu lieu comme prévu. Il est également vérifié par hybridation l'absence de tout fragment-détectable du ou des 2 marqueurs au sein du génome de la souche transformée notamment de tout fragment détectable du marqueur de contre-sélection, étant entendu que dans le cas de la construction du plasmide réplicatif d'expression, le marqueur positif est gardé, et en conséquence ce marqueur positif est choisi comme correspondant à une séquence d'ADN non exogène.

D'autres marqueurs de contre-sélection peuvent être utilisés parmi les gènes homologues ou hétérologues au génome de levure dont la surexpression conditionnelle en plasmide centromérique est toxique pour la levure hôte. On peut citer par exemple :
- *ATL2*, gène d'*Arabidopsis thaliana* codant pour une protéine à doigt de zinc (Martinez-Garcia et al. (1996) Gene isolation in *Arabidopsis thaliana* by conditional overexpression of cDNAs toxic to *Saccharomyces cerevisiae* : Identification of a novel early response zinc-finger gene. MGG, 252 :587-596.)
- *DUO1*, gène de *Saccharomyces cerevisiae* codant pour une protéine du cytosquelette (Hofmann et al. (1998) *Saccharomyces cerevisiae* Duolp and Dam1p, novel proteins involved in mitotic spindle function. J. Cell. Biol., 143 :1029-1040) ;
- *GIN1I*, gène de *Saccharomyces cerevisiae* codant pour une protéine subtélomérique (Kawahata et al. (1999) A positive selection for plasmid loss in *Saccharomyces cerevisiae* using galactose inducible growth inhibitory sequences. Yeast, 15 :1-10)
- *GIN12*/*SPC42*, gène de *Saccharomyces cerevisiae* codant pour une protéine du cytosquelette (Akada et al. (1997) Screening and identification of yeast sequences that cause growth inhibition when overexpressed. MGG, 254 :267-274)
- *H1-1*, gène d'*Arabidopsis thaliana* codant pour une histone H1 (Martinez-Garcia et al. (1996) Gene isolation in *Arabidopsis thaliana* by conditional overexpression of cDNAs toxic to *Saccharomyces cerevisiae* : Identification of a novel early response zinc-finger gene. MGG, 252 :587-596.)
- *TPK1*, gène de *Saccharomyces cerevisiae* codant pour une protéine kinase dépendante de l'AMPc (Liu, H. et al. (1992) Construction of a *GAL1*-regulated yeast cDNA expression library and its application to the identification of genes whose overexpression causes lethality in yeast. Genetics, 132 :665-673).

Les séquences répétées mosaïques de *Saccharomyces cerevisiae* correspondent, en général, à la construction d'une séquence d'environ 100 paires de bases constituée de fragments de gènes existant en principe dans la cellule hôte, chacun de ces fragments étant d'une longueur telle qu'il ne permet en principe aucun événement de recombinaison quand ils sont dans une séquence mosaïque. Ces séquences sont construites de telle manière qu'aucune chaîne peptidique ne peut être codée, par exemple grâce à l'introduction de codons stop à des endroits judicieux.

Les séquences mosaïques peuvent être construites à partir de courts fragments de gènes qui sont très probablement conservés dans les différentes souches de levure et situés dans des zones peu variables de ces gènes. Ces zones peu variables ont été déterminées par recherche d'homologie avec d'autres banques de gènes par le programme B.L.A.S.T.A. (Altschul *et al*., 1997, Nucleic Acids Res., 25, pp. 3389-3402). Les séquences introniques connues pour être très variables sont à éviter. De préférence quand la souche hôte appartient à l'espèce *Saccharomyces cerevisiae*, le choix des courts fragments composant la séquence mosaïque, est réalisé d'après la séquence de la souche de *Saccharomyces cerevisiae* du MIPS à Munich (Mewes *et al*., 1997, Nucleic Acids Res., 25, pp. 28-30). Ces fragments sont également choisis de manière à ce que la séquence mosaïque possède des codons non-sens, arrêtant la traduction dans les 6 cadres de lecture (3 cadres de lecture directe, et 3 cadres de lecture inverse).

Une des caractéristiques essentielles du module d'excision présent dans la cassette de transformation, objet de la présente invention, est qu'il peut être utilisé quel que soit le site d'intégration choisi dans le génome de la levure (cas de la cassette d'intégration/excision) ou quel que soit le ou les gènes d'intérêt à exprimer. Cette propriété est directement dépendante du choix des sites de restriction permettant d'introduire le ou les gènes d'intérêt et les séquences SR dans ladite cassette de transformation. La présence d'un site de restriction spécifique dans ces fragments d'ADN à introduire dans la cassette exclut que ledit site puisse être utilisé ensuite pour construire ladite cassette. Pour que le module d'excision puisse être utilisé aisément pour toute construction, il est nécessaire qu'il comporte à chacune de ses extrémités au moins un site de restriction dont la fréquence dans le génome est la plus faible possible. Une des caractéristiques de l'invention est que le module d'excision comporte au moins 3 sites rares à ses extrémités et de préférence au moins 5 sites rares de restriction. Un site rare de restriction est un site qui est reconnu par une endonucléase de type II reconnaissant une séquence octanucléotidique ou tout site de restriction ayant la même caractéristique de rareté. Pour illustrer cette notion de fréquence rare, il est rappelé que dans une séquence d'ADN ayant une composition 50%A-T et 50% G-C, la fréquence de la présence d'un hexanucléotide spécifique est de 1 pour 4096 et d'un octanucléotide spécifique est 1 pour 65536. Par exemple, le module d'excision aura à son extrémité 5', les sites *Pac*I, *Asc*I et *Pme*I, et à son extrémité 3' les sites *Fse*I et *Swa*I.

La présente invention concerne également un procédé d'intégration d'un gène d'intérêt ou d'inactivation d'un gène dans une levure, caractérisé en ce que :
a) on transforme ladite levure à l'aide d'une construction d'ADN consistant en la cassette d'intégration/excision telle que décrite précédemment,
b) on sélectionne les levures ayant intégré ladite cassette grâce au marqueur positif,
c) puis, on sélectionne parmi ces levures les levures dans lesquelles la cassette a été excisée grâce aux séquences SRD en recherchant les levures dépourvues du marqueur négatif.

Plus particulièrement, l'invention concerne un procédé d'intégration de plusieurs copies ou d'inactivation des différentes copies d'un gène dans une levure, caractérisé en ce qu'on répète le procédé décrit précédemment avec une cassette d'intégration/excision qui comporte pour chaque répétition du procédé au moins une séquence d'ADN recombinogénique différente choisie de façon à ce que chaque site comportant une intégration ne puisse pas, en principe, faire l'objet d'une recombinaison avec la cassette suivante.

Les cassettes d'intégration/excision comportant éventuellement un gène d'intérêt correspondant aux définitions données ci-dessus peuvent être construites selon les méthodes usuelles utilisées en la matière par l'homme de l'art.

Il en est de même du plasmide navette *E. coli-S.cerevisiae* permettant d'obtenir après excision des éléments de construction un plasmide réplicatif ou multicopie d'expression dans la levure, sans ADN exogène autre que celui du ou des gènes d'intérêt. Les principes de construction, les éléments de ce plasmide navette sont les mêmes que ceux ci-dessus exposés.

Plus particulièrement, l'invention concerne aussi un procédé de transformation d'une levure auxotrophe afin d'obtenir un nombre élevé de copies d'au moins un gène d'intérêt avec un plasmide navette comme défini ci-dessus dont le marqueur de sélection ou marqueur positif d'origine levure est un marqueur de complémentation de l'auxotrophie de la levure hôte, caractérisé en ce que :
- on transforme la levure avec ledit plasmide navette ;
- on sélectionne sur milieu minimal, c'est à dire sur un milieu ne comportant pas l'élément pour lequel la levure est auxotrophe, permettant l'induction du marqueur dominant négatif présent dans la région du plasmide navette comportant les fragments d'ADN d'*E. coli* délimitée par les 2 séquences SRD, les cellules ne portant que des plasmides dont la partie correspondant aux fragments d'*E. coli* et au marqueur négatif a été excisée.

Parmi les procédés de transformation utilisables des souches de levure, on peut citer notamment celui proposé par Ito *et al*. (1983, J. Bacteriol., vol. 153, pp. 163-168), ou par Klebe *et al*. (1983, Gene, vol. 25, pp. 333-341), ou encore par Gysler *et al*., (1990, Biotechn. Techn., vol. 4, pp. 285-290).

Enfin, la présente invention concerne les levures transformées par une cassette selon l'invention et obtenues par un procédé tel que décrit précédemment et qui ne contient que de l'ADN de levure, à l'exception éventuellement du ou des gènes codants pour une protéine d'intérêt, les séquences SRD étant non codantes. Si la cassette selon l'invention est utilisée pour inactiver une famille de gènes, les levures selon la présente invention seront telles que le nombre désiré de copies, c'est à dire le nombre désiré d'allèles de la même famille de gènes, aura été inactivé par intégration/excision à l'aide de cassettes selon la présente invention, ou éventuellement l'ensemble des copies aura été inactivé, de manière à choisir avec quelle intensité un gène sera exprimé. De même, si une des cassettes selon l'invention est utilisée pour exprimer un ou plusieurs gènes d'intérêt, le nombre désiré de copies de ce gène pourra être obtenu.

Parmi les levures plus particulièrement intéressantes selon la présente invention, il faut citer les levures du genre Saccharomyces et notamment *Saccharomyces cerevisiae*, en particulier les souches industrielles et plus particulièrement celles de levures de panification.

### LISTE DES FIGURES EN ANNEXE

Figure 1: Composition d'une séquence répétée directe mosaïque.
Figure 2: Plasmide pFA6-kanMX4, portant le marqueur de sélection dominant kanMX et servant de base à la construction d'une cassette d'intégration.
Figure 3: Stratégie de clonage de deux séquences répétées directes mosaïques dans le plasmide pFA6-kanMX4.
Figure 4: Plasmide pMOS-5', résultant de l'ajout d'une séquence répétée directe mosaïque dans le plasmide pFA6-kanMX4 en 5' du marqueur kanMX.
Figure 5: Plasmide pMOS-3' résultant de l'ajout d'une séquence répétée directe mosaïque dans le plasmide pFA6-kanMX4 en 3' du marqueur kanMX.
Figure 6: Plasmide pMOS, résultant de la combinaison du plasmide pMOS-5' et du plasmide pMOS-3', portant le marqueur de sélection kanMX et les deux séquences répétées directes mosaïques permettant la perte du module d'excision par recombinaison homologue.
Figure 7: Plasmide pYEDP-60.2-RTA, portant le module suicide RTA à intégrer dans le plasmide pMOS et qui agira en tant que marqueur de sélection négative dans la cassette d'intégration.
Figure 8: Plasmide pINT-MOS, portant la partie centrale dite "module d'excision" de la cassette d'intégration.
Figure 9: Plasmide pMEL-5', résultant de l'ajout d'une première séquence répétée directe MEL dans le plasmide pFA6-kanMX4 en 5' du marqueur kanMX.
Figure 10: Plasmide pMEL, résultant de l'ajout d'une deuxième séquence répétée directe MEL dans le plasmide pMEL-5'.
Figure 11: Plasmide pINT-MEL, portant le marqueur de sélection kanMX, deux séquences répétées directes MEL, le module suicide RTA.
Figure 12: Plasmide pSUC-A, portant la cassette de disruption CAS-SUC-A d'un allèle du gène *SUC* dans *Saccharomyces cerevisiae*.
Figure 13 : Plasmide pSE936-H1-1, portant l'ADNc du gène *H1-1*, utilisé comme marqueur négatif alternatif à *RTA*.
Figure 14 : Plasmide pINT-MOS-H1-1, portant le module d'excision dont la région codante du gène *RTA* est remplacée par l'ADNc du gène *H1-1*.
Figure 15 : Plasmide pURA, portant la cassette CAS-URA de délétion du gène *URA3*.
Figure 16 : Plasmide pJB202, résultant du remplacement des marqueurs de sélection positif et négatif du pINT-MOS par un oligonucléotide linker contenant les sites uniques de restriction *EcoRI, AscI*, *PmeI*, *SwaI*, *PacI et HindIII*.
Figure 17 : Plasmide pJB205 résultant de l'ajout successif du gène *URA3*, puis du fragment 2 micron enfin du module RTA dans le plasmide pJB202.
Figure 18: Plasmide pJB206 résultant de l'ajout de la cassette d'expression du gène *ldhL* dans le plasmide pJB205.
Figure 19 : Plasmide pJB206-OUT résultant de l'excision du fragment contenant le marqueur négatif et l'ADN d'origine *E. coli* hors du plasmide pJB206.

### Liste de séquences

SEQ ID N°1: la séquence SEQ ID N°1 est la séquence répétée directe mosaïque dont la conception est décrite dans l'exemple N° 1 de la présente invention.
SEQ ID N°2: la séquence SEQ ID N°2 est la séquence répétée directe MEL utilisée dans l'exemple N° 3 de la présente invention.
SEQ ID N°3 : séquence d'ADN de liaison ou linker utilisée dans l'exemple 7 pour introduire des sites de restriction.
SEQ ID N°4 : second linker utilisé dans l'exemple 7.

### EXEMPLES

### EXEMPLE 1 : composition d'une séquence répétée directe dite "mosaïque"

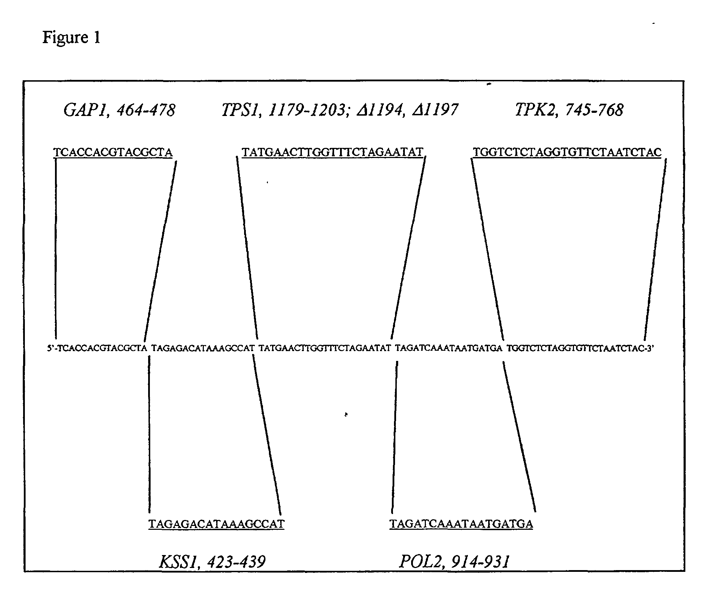

La séquence répétée directe mosaïque, non hétérologue de *Saccharomyces cerevisiae* est conçue selon la démarche présentée dans la description, en l'occurrence elle comporte les 5 séquences codantes suivantes correspondant à un total de 97 paires de bases (Figure 1). Les fragments sont choisis de manière à ce que la séquence mosaïque possède des codons stop empêchant la traduction dans les 6 cadres de lecture. La position des fragments d'ADN sélectionnés pour la mosaïque est donnée par référence au codon de début de traduction selon la classification du MIPS (Munich Information Center for Protein Sequence) :
- *GAP1*, pb 464-478, provenant de l'ORF de YKR039W, codant pour une glycoprotéine de membrane servant au transport des acides aminés,
- *KSS1*, pb 423-439, provenant de l'ORF de YGR040W, codant pour une protéine homologue d'une MAP kinase,
- *TPS1*, pb 1179-1203, provenant de l'ORF de YBR126C, codant pour la tréhalose-phosphate synthétase ; deux résidus cytosine en position 1194 et 1197 sont supprimés dans ce fragment de manière à générer un site de restriction *Xba*I,
- *POL2,* pb 914-931, provenant de l'ORF de YNL262W, codant pour la grande sous-unité epsilon de l'ADN-polymérase,
- *TPK2,* pb 745-768, provenant de l'ORF de YPL203W, codant pour une sous-unité catalytique de la protéine kinase AMPc dépendante.

La séquence mosaïque résultant de l'assemblage des fragments ci-dessus identifiés est donnée comme SEQUENCE ID N°1.

L'absence pratique d'homologie entre le génome de *Saccharomyces cerevisiae* et la séquence mosaïque présentée en SEQUENCE ID N°1 a été contrôlée à l'aide d'un programme classique tel que BLAST search version BLASTN 2.0.5 accessible sur Internet (référence Altschul *et al*., 1997, Nucleic Acids Res., vol. 25, pp. 3389-3402). Il a été mis en évidence qu'il n'existait aucune séquence génomique proche de cette séquence mosaïque, c'est-à-dire que le degré d'identité de la séquence mosaïque totale avec une partie quelconque du génome est inférieure à 25%, donc suffisamment faible pour exclure l'éventualité d'une recombinaison homologue entre un fragment d'ADN de *Saccharomyces cerevisiae* et la séquence mosaïque.

### EXEMPLE 2 : Construction d'un module d'excision avec séquences mosaïques

Dans cet exemple, les deux séquences répétées directes permettant l'excision du module par recombinaison homologue sont la séquence mosaïque définie dans l'exemple N°1 ci-dessus.

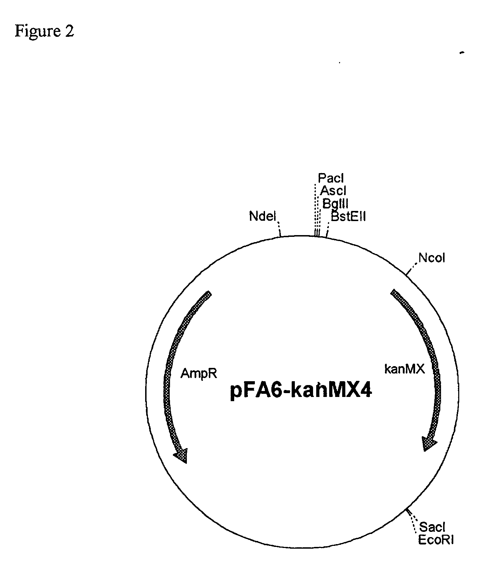

Les éléments du module d'excision sont construits dans l'ordre suivant :
1) le plasmide pFA6-kanMX4 portant le marqueur de sélection dominant kanMX, sert de base à la construction (figure 2);
2) les séquences répétées directes mosaïques sont ajoutées séparément dans le plasmide pFA6-kanMX4, puis combinées dans un seul plasmide;
3) le gène suicide *RTA* sous contrôle du promoteur *GAL10-CYC1* est inséré ;

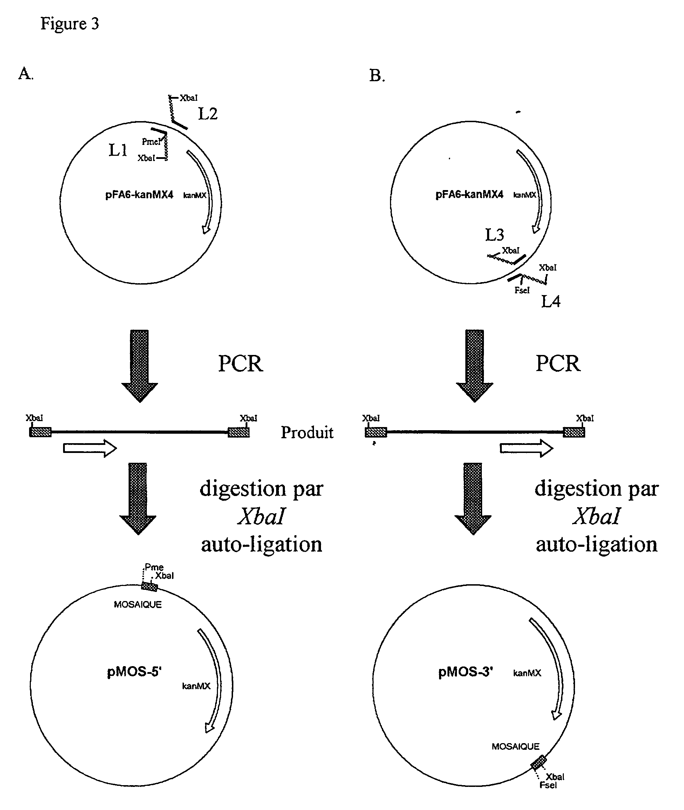

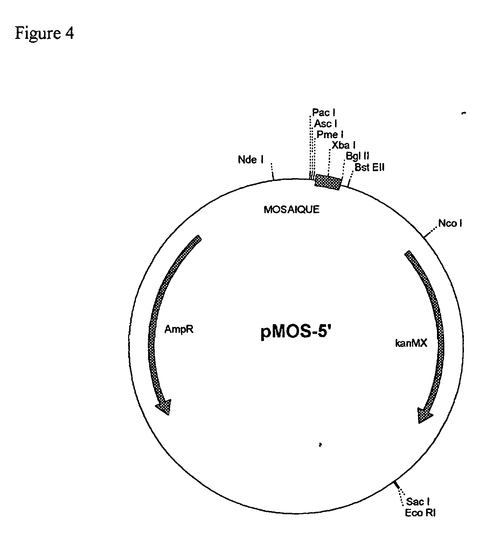

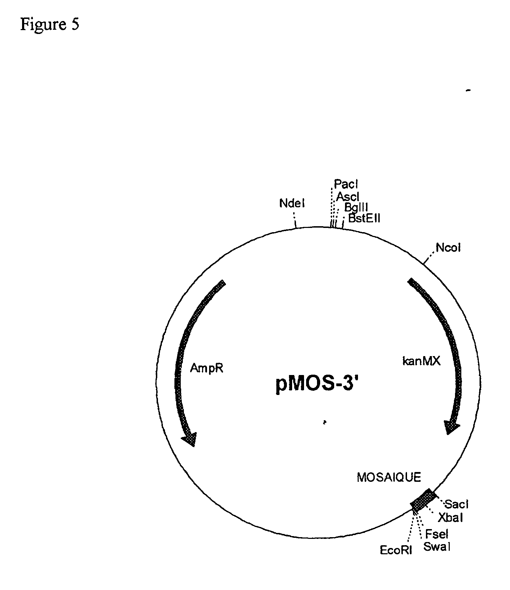

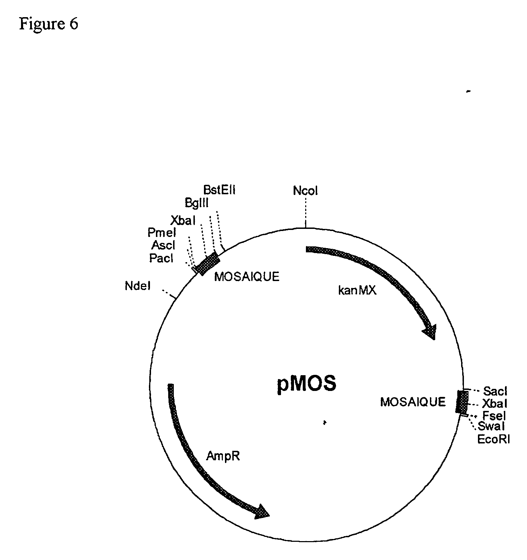

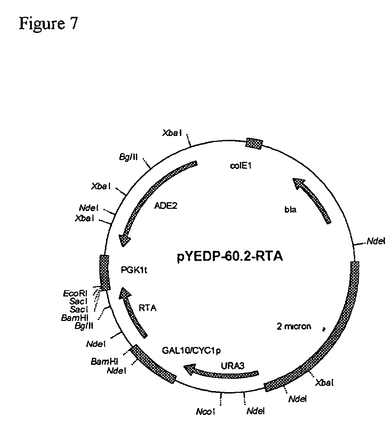

Chacune de ces étapes est réalisée de préférence de la manière suivante :
1) Le marqueur kanMX est porté par le plasmide pFA6-kanMX4. Ce marqueur est composé de la partie codante du gène *kan*^{*r*} conférant la résistance à la kanamycine, sous contrôle d'un promoteur et d'un terminateur. Le gène kan^{r}, issu du transposon Tn 903 d'*Escherichia coli*, code une aminoglycoside phosphotranférase responsable de la résistance à la kanamycine. Le promoteur et le terminateur sont les séquences de contrôle du gène *TEF* (Translation Elongation Factor) du champignon filamenteux *Ashbya gossypii*. La construction du plasmide pFA6-kanMX4 est décrite en détail dans l'article de Wach *et al*. (Yeast, 1994, 10, pp.1793-1808) ou dans l'ouvrage Methods in Microbiology (vol. 26, Yeast Gene Analysis, chap. 5, pp. 67-81, ACADEMIC PRESS, ISBN 0-12-521526-6). On peut aussi utiliser tout promoteur et terminateur fonctionnant dans *Saccharomyces cerevisiae* et ayant une faible homologie avec les promoteurs et terminateurs de *Saccharomyces cerevisiae*. Une autre solution pour éviter des recombinaisons non désirées dans *Saccharomyces cerevisiae* est de faire une combinaison promoteur et terminateur provenant de gènes différents de *Saccharomyces cerevisiae*, de préférence le promoteur et/ou le terminateur étant hybrides comme le promoteur *GAL10-CYC1*.
2) L'insertion dans le plasmide pFA6-kanMX4 des séquences mosaïques de *Saccharomyces cerevisiae* en amont et en aval du marqueur kanMX est réalisée selon la stratégie suivante illustrée par la figure 3 :
   La séquence mosaïque référencée SEQ ID N°1 est intégrée du côté 5' de kanMX entre les sites de restriction *Asc*I et *Bgl*II du plasmide pFA6-kanMX4 donnant un plasmide intermédiaire pMOS-5' (figure 4).
   En parallèle la séquence mosaïque référencée SEQ ID N°1 est intégrée du côté 3' de kanMX entre les sites de restriction *Sac*I et *Eco*RI dans le plasmide pFA6-kanMX4 donnant un plasmide intermédiaire pMOS-3' (Figure 5).
   Puis ces deux plasmides sont digérés et liés de manière à obtenir le plasmide pMOS portant les deux séquences mosaïques de part et d'autre du marqueur kanMX (figure 6).
   Pour l'insertion de la séquence mosaïque du côté 5' de kanMX (figure 3A), le plasmide pFA6-kanMX4 est amplifié par PCR inverse (dans une PCR inverse, c'est le plasmide dans son intégralité qui est amplifié en tant qu'ADN matriciel). La fusion de la séquence mosaïque avec le plasmide est faite à l'aide de deux amorces L1 et L2 composées chacune d'environ 70 à 90 bases, à savoir une cinquantaine de bases de la séquence mosaïque accolée à un segment d'une vingtaine de bases destiné à s'hybrider au segment complémentaire de pFA6-kanMX4.
   Autrement dit l'amorce L1 est composée de la séquence complémentaire à la première cinquantaine de bases de la séquence mosaïque, correspondant à environ la moitié de ladite séquence mosaïque et d'une vingtaine de bases correspondant à la séquence du plasmide en 5' du point d'insertion. L'amorce L2 est composée de la seconde cinquantaine de bases correspondant à la deuxième moitié de la séquence mosaïque référencée SEQ ID N°1 et d'une vingtaine de bases correspondant à la séquence du plasmide en 3' du point d'insertion. L'amorce L1 contient en plus entre la demi séquence mosaïque et les bases s'hybridant au plasmide, la séquence du site de restriction *Pme*I qui permettra éventuellement l'ajout ultérieur d'un gène d'intérêt spécifiquement au niveau de ce site. Ces deux amorces L1 et L2 portent à leurs extrémités le site de restriction *Xba*I défini à l'exemple 1.
   Ces deux amorces L1 et L2 sont préparées par synthèse in vitro d'oligonucléotides et purifiées. Ces synthèses d'amorces pures sont réalisées par des sociétés spécialisées. Sauf mention contraire, toutes les amorces définies ci-après sont obtenues de la même manière.
   L'amplification par PCR inverse du plasmide pFA6-kanMX4 en présence des deux amorces L1 et L2 est réalisée selon les recommandations de PCR Protocols, a Guide to Methods and Applications, M.A. Innis *et al*., Academic Press Inc., 1990, Part II, pp. 219-227.
   Cette réaction PCR est catalysée par une polymérase thermophile générant des bouts francs, ladite polymérase étant de préférence dépourvue d'activité exonucléase, par exemple cette enzyme peut être la Vent_{R}®(exo⁻)DNA polymérase commercialisée par New England Biolabs, Inc., USA. Le milieu réactionnel est le milieu classique préconisé par le fournisseur.
   Les échantillons obtenus par PCR sont ensuite séparés par migration dans un gel d'agarose et le fragment correspondant à la séquence d'ADN recherchée est isolé et élué à l'aide du kit Qiaquick (Qiagen GmbH, Allemagne) selon le protocole indiqué par le fabricant.
   Le fragment purifié ainsi obtenu est cyclisé par une technique appropriée, pour obtenir un plasmide circulaire appelé pMOS-5'(figure 4). Une méthode consiste à faire apparaître des extrémités cohésives aux extrémités du fragment obtenu par PCR. Pour cela, on utilise le site de restriction *Xba*I présent à l'extrémité 5' de chaque amorce L1 et L2. Le produit PCR obtenu par amplification contient alors ce site à chaque extrémité des fragments linéaires. Une digestion par l'enzyme de restriction *Xba*I conduit à la création d'extrémités cohésives qui favorisent la cyclisation.
   Parallèlement, la séquence mosaïque est insérée dans le plasmide pFA6-kanMX4 du côté 3' du marqueur kanMX (figure 3B). Le plasmide pFA6-kanMX4 est amplifié par PCR inverse en présence de deux amorces, L3 et L4. Ces amorces comprennent chacune une moitié de la séquence mosaïque correspondant à une cinquantaine de bases, liées à une vingtaine de bases permettant l'hybridation en 5' et en 3' du point d'insertion compris entre les sites *Sac*I et *Eco*RI du plasmide pFA6-kanMX4. Les amorces L3 et L4 sont composées selon le même principe que les amorces L1 et L2, c'est-à-dire qu'elles contiennent respectivement la séquence complémentaire à la première moitié et la seconde moitié de la séquence mosaïque, soit une cinquantaine de bases chacune, et une vingtaine de bases s'hybridant au plasmide l'une en aval, l'autre en amont du point d'insertion. Dans l'amorce L4, est ajouté en outre un site de restriction qui permettra l'intégration ultérieure d'une séquence recombinogénique SR. Ce site de restriction, par exemple *Fse*I, est inséré entre la demi séquence mosaïque et les bases s'hybridant au plasmide. Après amplification du plasmide pFA6-kanMX4 en présence des amorces L3 et L4 par PCR inverse selon des techniques déjà citées pour les amorces L1 et L2, le produit PCR est purifié, puis cyclisé par une méthode appropriée, par exemple celle proposée ci-dessus pour la cyclisation de pMOS-5'. Le site de restriction *Swa*I est alors introduit entre d'une part le site *Eco*RI présent dans le plasmide et d'autre part le site *Fse*I porté par la séquence mosaïque, grâce à un linker (fragment de liaison) portant le site *Swa*I en son centre entouré des extrémités cohésives *Eco*RI et *Fse*I.
   Le plasmide résultant de l'ensemble de ces constructions est appelé pMOS-3' (figure 5).
   Finalement, les plasmides pMOS-3' et pMOS-5' sont digérés par des enzymes de restriction appropriées, par exemple par les endonucléases *Nde*I et *Nco*I, afin que les fragments complémentaires soient liés. Le plasmide généré est linéarisé par *Bam*HI, traité par la Mung Bean Nuclease *(New England Biolabs U.K.)* et recircularisé sur lui même afin d'éliminer le site de restriction *Bam*HI, pour obtenir le plasmide pMOS (figure 6) portant une séquence mosaïque complète de chaque côté du marqueur kanMX.
   Les deux séquences répétées mosaïques portées par pMOS sont la séquence référencée SEQ ID N°1.
   Le plasmide pMOS contient 5 sites rares dé restriction, à savoir les sites *Pme*I, *Fse*I et *Swa*I apportés dans le cadre des constructions décrites ci-dessus, plus deux sites rares à savoir *Pac*I et *Asc*I portés initialement par le plasmide pFA6-kanMX4 dont on est parti à titre d'exemple pour les constructions. Bien entendu, si les sites rares nécessaires ne sont pas présents dans le plasmide de départ, ils peuvent être ajoutés à l'aide d'un linker.
3) Introduction du module suicide RTA dans le module d'excision porté par le plasmide pMOS:
   Le module suicide RTA est porté par le plasmide pYEDP-60.2-RTA décrit en figure 7 et déposé aux termes du Traité de Budapest dans la souche d'*Escherichia coli* DH5α[YEDP-60.2-RTA] à la Collection Nationale de Culture de Micro-organismes, C.N.C.M., Institut Pasteur, 25 rue du Docteur Roux, 75724 Paris cedex 15, sous le n° I-2083 le 1er octobre 1998. Ce module suicide est composé :
   - de la partie codante d'un gène codant pour un composé toxique pour la levure
   - d'un promoteur hybride inductible de *Saccharomyces cerevisiae*, ce promoteur hybride ayant été choisi pour ses caractéristiques de promoteur fort, pratiquement inactif en dehors du milieu d'induction, ce qui permet aux souches de ne pas être gênées dans leur croissance par une activité résiduelle du promoteur. Le promoteur hybride *GAL10-CYC1* est décrit dans l'article de L. Guarente *et al*, 1982, Proc. Natl. Acad. Sci. USA, vol.79, pp. 7410-7414.
   - d'un terminateur provenant d'un autre gêne de *Saccharomyces cerevisiae*.

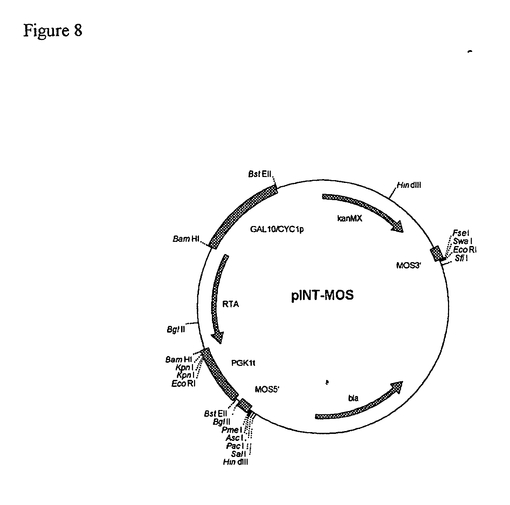

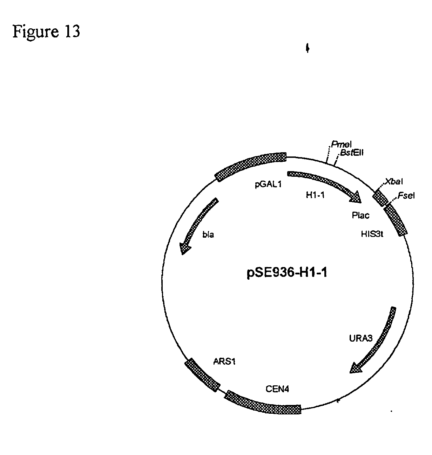

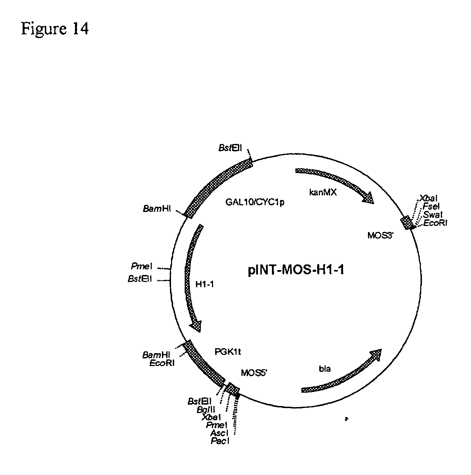

   Une telle construction a le mérite de minimiser les risques de recombinaison non désirée.
   Le plasmide pYEDP-60.2-RTA est ensuite employé pour introduire le module suicide RTA au niveau du site de restriction *Bst*EII du plasmide pMOS, site initialement présent dans le plasmide pFA6-kanMX4. Pour ce faire, le module suicide RTA est amplifié par PCR en utilisant le plasmide pYEDP-60.2-RTA comme matrice et en présence des amorces L5 et L6. Les amorces L5 et L6 sont conçues de manière à introduire des sites de restriction *Bst*EII aux deux extrémités du module suicide RTA. La réaction de PCR est réalisée selon les techniques classiques décrites par Sambrook et *al*., (Molecular Cloning, a Laboratory Manual, Cold Spring Harbor Laboratory Press, 1989, vol. 2, pp. 14.01-14.35). De même les amplifications par PCR des différents fragments utilisés dans la présente invention sont réalisées sauf mention contraire selon les recommandations de cet ouvrage. Après digestion par l'enzyme *Bst*EII, le module suicide RTA est inséré dans le plasmide pMOS, préalablement rendu linéaire par digestion avec *BstEII*. Le plasmide ainsi obtenu est appelé pINT-MOS (figure 8).
*4*) Remplacement du gène suicide *RTA* porté par le plasmide pINT-MOS par le gène *H1-1*.
   Un autre candidat particulièrement intéressant comme marqueur négatif est le gène *H1-1* d'*Arabidopsis thaliana*. Ce gène code pour une histone, c'est-à-dire une protéine constitutive de la chromatine des organismes supérieurs, son mode d'action ne semble pas être lié à une quelconque toxicité intrinsèque, mais à sa surexpression qui désorganise le noyau. Il est particulièrement intéressant pour limiter toute prévention, d'employer comme marqueur négatif un gène codant pour une protéine qui n'a pas de toxicité intrinsèque, comme une histone, mais dont la surexpression est capable de désorganiser une fonction de la cellule et d'inhiber en conséquence sa croissance.
   L'ADNc du gène *H1-1* est porté par le plasmide pSE936-H1-1 (Martinez-Garcia et al. (1996) Gene isolation in *Arabidopsis thaliana* by conditional overexpression of cDNAs toxic to *Saccharomyces cerevisiae* : Identification of a novel early response zinc-finger gene. MGG, 252 :587-596) donné figure 13. Le plasmide pSE936-H1-1 porte l'origine de réplication ColE1, le promoteur lac et le gène bla nécessaires aux réplication, expression et sélection dans *E. coli*, ainsi que le marqueur *URA3*, l'origine de réplication ARS1 stabilisée par le centromère CEN4 et la cassette d'expression d'ADNc composée du promoteur *GAL1* et du terminateur *HIS3* nécessaires aux réplication, expression et sélection dans *Saccharomyces cerevisiae*. (Elledge *et al., 1991, λYES :* A multifonctional cDNA expression vector for the isolation of genes by complementation of yeast and *E. coli* mutations, Proc. Natl. Acad. Sci. USA, vol. 88, pp. 1731-1735). La séquence de l'ADNc de *H1-1* contenu dans le pSE936-*H1-1* peut être déduite de la séquence du gène *H1-1* déposée auprès de GenBank par S. Gantt (S. GANTT et T.R. LENVIK (1991) *Arabidopsis thaliana* H1 Histones : Analysis of two members of a small gene family. Eur. J. BIOCHEM. 202 :1029-1039) accessible sous le N°X62458. L'ADNc du gène *H1-1* est amplifié par PCR à l'aide des amorces H1 et H2 introduisant un site *Bam*HI aux extrémités 5' et 3' du produit de PCR. Le produit de PCR correspondant à *H1-1* est digéré par *Bam*HI et cloné dans le site *Bam*HI du pINT-MOS en remplacement de *RTA*, donnant le pINT-MOS-H1-1 (figure 14).
5) Introduction d'un gène d'expression dans le plasmide intégratif pINT-MOS Cette dernière étape ne sera bien entendu réalisée que dans le cas où un gène d'intérêt doit être introduit dans le génome. Pour ce faire, le gène est inséré au niveau du site de restriction *Pme*I ou *Asc*I ou *Fse*I présents dans le plasmide pINT-MOS et/ou au niveau du site de restriction *Asc*I ou *Fse*I présents dans le plasmide pINT-MOS-H1-1, dans lequel le site de restriction *Pme*I n'est plus utilisable car aussi présent dans le marqueur *H1-1*.

### EXEMPLE 3 : Construction d'un module d'excision avec séquences SRD MEL

Dans cet exemple, les deux séquences répétées directes permettant l'excision du module par recombinaison homologue sont des fragments issus du gène *MEL1*.

Le gène *MEL1* est le gène de *Saccharomyces cerevisiae* variété *carlsbergensis* codant une alpha-galactosidase qui dégrade le mélibiose. *MEL1* est absent dans les levures de boulangerie, ce qui évite toute recombinaison éventuelle entre les séquences SRD MEL et des séquences du génome des souches de levures de panification. Les séquences SRD MEL sont constituées d'une séquence inférieure ou égale à 300 pb choisie dans la partie codante du gène *MEL1* et comportant de préférence des codons stop positionnés dans les 6 cadres de lecture de la traduction. Ainsi, toute traduction de la séquence de *MEL1* choisie en un polypeptide chimère est évitée.

Ledit module d'excision avec séquences SRD MEL peut être construit par n'importe quelle stratégie permettant de répondre aux exigences ci-dessus énoncées.

Selon un mode de réalisation particulier, ledit module d'excision est construit de la manière suivante:
1) le plasmide pFA6-kanMX4 portant le marqueur de sélection dominant kanMX sert de base à la construction du module d'excision.
2) les séquences SRD MEL sont ajoutées successivement dans le plasmide pFA6-kanMX4 ;
3) le module suicide RTA est inséré.

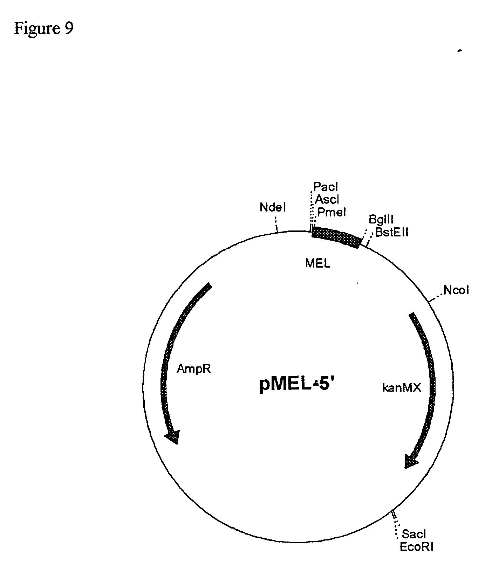

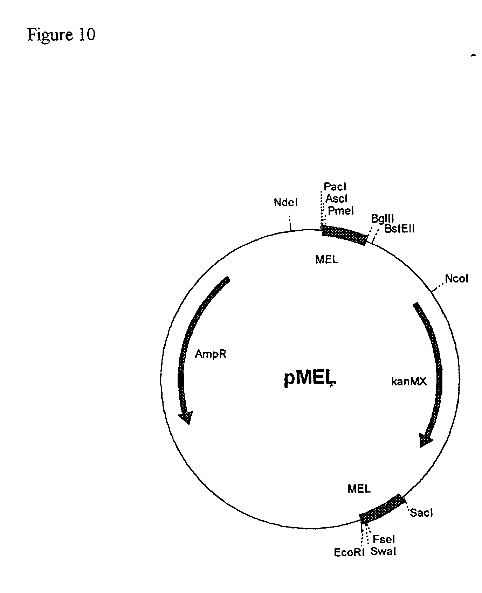

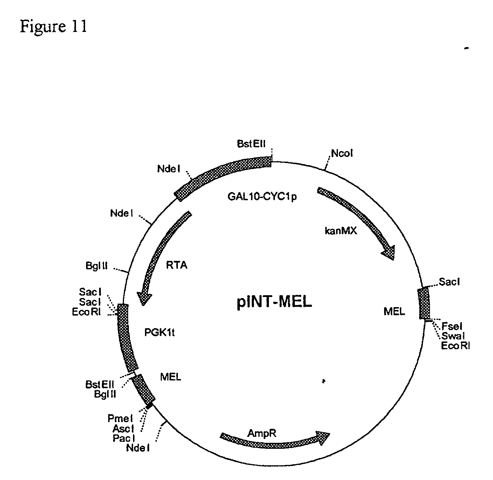

Chacune de ces étapes est réalisée de la manière suivante:
1) le plasmide pFA6-kanMX4 servant de base à la construction est le même plasmide que celui utilisé dans l'exemple 2 et représenté figure 2.
2) Les séquences SRD MEL sont insérées de part et d'autre du marqueur kanMX dans le plasmide pFA6-kanMX4, de la manière suivante:
   La séquence répétée directe MEL choisie est un fragment d'ADN de 209 pb situé dans la région codante du gène *MEL1*, comme par exemple le fragment représenté par la séquence SEQ ID N°2.
   La première copie de la séquence SRD MEL est introduite en 5' du marqueur kanMX, donnant le plasmide pMEL-5' (figure 9). Pour cela, la séquence SRD MEL est amplifiée par PCR en utilisant de l'ADN génomique de *Saccharomyces carlsbergensis* comme matrice, à l'aide de deux amorces, L11 et L12. L'amorce L11 porte les sites de restriction *Asc*I et *Pme*I, l'amorce L12 porte le site de restriction *Bgl*II. Ainsi, lors de la PCR, les sites de restriction *Asc*I et *Pme*I sont introduits en 5' de la séquence SRD MEL et le site de restriction *Bgl*II est introduit en 3' de la séquence SRD MEL. Le produit de la PCR est purifié et digéré avec les enzymes *Asc*I et *Bgl*II. Le plasmide pFA6-kanMX4 a été également digéré avec ces mêmes enzymes. Les deux fragments ainsi obtenus sont liés donnant le plasmide pMEL-5' (figure 9).
   Puis la seconde copie de la séquence SRD MEL est ajoutée dans le plasmide pMEL-5', en 3' du marqueur kanMX. Pour cela la séquence SRD MEL est amplifiée par PCR en utilisant de l'ADN génomique de *Saccharomyces carlsbergensis* comme matrice en présence de deux amorces, L13 et L14. L'amorce L13 porte le site de restriction *Sac*I, l'amorce L14 porte les sites de restriction *Fse*I, *Swa*I et *Eco*RI. Ainsi lors de la PCR, le site de restriction *Sac*I est introduit en 5'de la séquence SRD et les sites de restriction *Fse*I, *Swa*I et *Eco*RI sont introduits en 3' de la séquence SRD MEL, le site *Eco*RI étant situé à l'extrémité 3' de la séquence SRD. Le produit de PCR a été purifié et digéré par les enzymes *Sac*I et *Eco*RI et introduit dans le plasmide pMEL-5', préalablement digéré avec les mêmes enzymes. On obtient alors le plasmide pMEL (figure 10).
3) L'introduction du gène *RTA* est réalisée de la même manière que dans l'exemple 2 point 3) ci-dessus. Le plasmide obtenu est appelé pINT-MEL (figure 11).

### EXEMPLE 4 : Délétion des différents allèles du gène SUC dans Saccharomyces cerevisiae

*Saccharomyces cerevisiae* possède plusieurs gènes dispersés dans le génome codant des enzymes ayant la même activité invertase, appelés *SUC1*, *SUC2,* etc.... Dans les souches de levure de panification, cette activité invertase est parfois gênante et il est utile de la réguler. Pour réduire le taux d'invertase produite, un certain nombre de gènes *SUC* responsables de sa production doivent être inactivés à l'aide de différentes cassettes d'intégration/excision. Pour cela, le module d'excision selon l'invention est assemblé entre des séquences recombinogéniques (séquences SR) capables de se fixer sur le site chromosomique visé et de s'intégrer par recombinaison avec des séquences homologues présentes dans les gènes *SUC* de *Saccharomyces cerevisiae*. Différentes cassettes d'intégration/excision appelées CAS-SUC-N, avec N = A, B, C,..., (ces lettres précisant l'ordre d'utilisation de cassettes d'intégration) sont construites puis mises en oeuvre successivement jusqu'à obtenir l'activité invertase requise.

### A) Principe de construction des cassettes d'intégration/disruption CAS-SUC

Une cassette CAS-SUC est composée d'un module d'excision et des deux séquences recombinogéniques SR choisies dans une région présente dans l'ensemble des loci SUC de manière à ce que la double recombinaison homologue conduisant à l'intégration de la cassette puisse se réaliser dans n'importe quel locus SUC du génome.

Par exemple, pour réaliser 3 disruptions, 3 cassettes sont construites à partir d'un plasmide portant le module d'excision décrit dans l'exemple 2 ci-dessus, c'est-à-dire le module d'excision porté par le plasmide pINT-MOS. Pour les 3 cassettes, la première séquence SR située à l'une des extrémités de la cassette d'intégration est la même et est appelée SUC-5'. Ce fragment d'environ 200 pb est homologue à la région 5' de la séquence codante du gène *SUC2.* La deuxième séquence SR située à l'extrémité opposée de la cassette d'intégration change pour chacun des 3 événements d'intégration désirés et différencie les trois cassettes. Ce sont trois fragments d'environ 200 pb choisis dans la région 3' de la séquence codante du gène *SUC2,* en partant de l'extrémité 3' de cette séquence et en remontant vers la région 5'. En d'autres termes, pour la première intégration, le fragment le plus externe appelé SUC-3'-A est utilisé en tant que séquence recombinogénique de pair avec le fragment SUC-5'; l'intégration suivante est réalisée à l'aide du fragment médian SUC-3'-B et du fragment SUC-5', et la dernière intégration est réalisée avec le fragment le plus interne SUC-3'-C et le fragment SUC-5'. Les différentes séquences recombinogéniques SR choisies ne doivent pas être homologues entre elles. Les séquences SR décrites ci-dessus peuvent être déterminées aisément à partir de la séquence du gène *SUC2,* référencée YI9402.12 dans la banque de données du MIPS. Les trois cassettes ainsi obtenues sont appelées CAS-SUC-A, CAS-SUC-B et CAS-SUC-C.

Les 3 variantes de la cassette CAS-SUC sont issues du plasmide pINT-MOS dans lequel les fragments recombinogéniques sont insérés de part et d'autre du module d'excision. Pour ce faire les fragments SUC-5', SUC-3'-A, SUC-3'-B, SUC-3'-C sont amplifiés par PCR en utilisant de l'ADN génomique de la souche de laboratoire CEN.PK122 (EUROSCARF Strain Collection, Allemagne). Lors de la PCR, un site de restriction *Pac*I est introduit en 5' du fragment SUC-5' et deux codons STOP ainsi que le site de restriction *Asc*I sont introduits en 3' du fragment SUC-5'. De même, les 3 fragments SUC-3'-A, SUC-3'-B et SUC-3'-C amplifiés par PCR sont flanqués en 5' du site de restriction *Fse*I et en 3' du site de restriction *Swa*I. Ces divers sites de restriction permettront l'insertion des séquences SR dans le plasmide pINT-MOS au niveau des sites correspondants. Les codons STOP introduits dans le cadre de lecture du gène *SUC2* empêchent une éventuelle traduction de la séquence créée par la juxtaposition d'un segment du gène *SUC2* et du premier segment formant la séquence mosaïque. Le risque de synthétiser une protéine chimère est ainsi écarté.

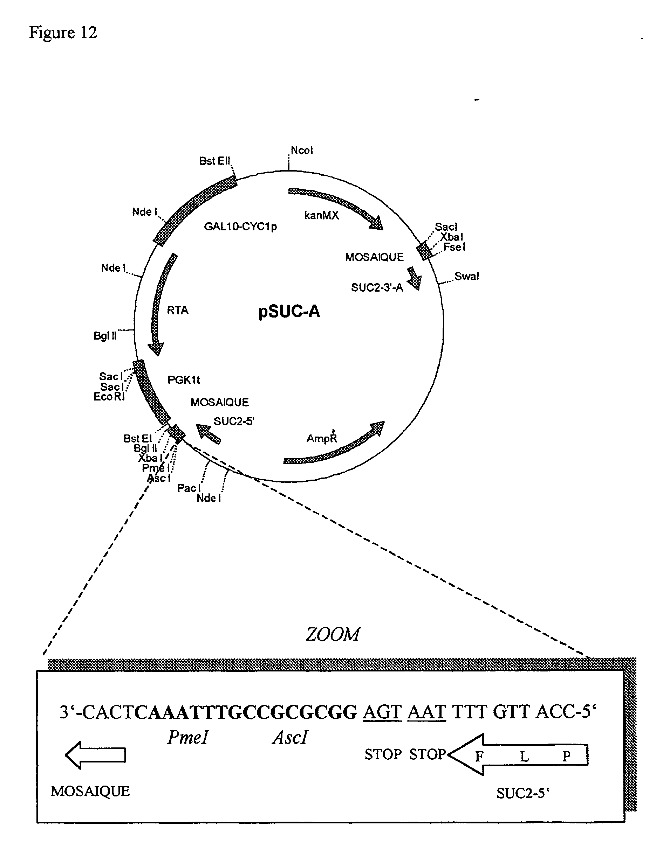

La séquence recombinogénique SUC-5' obtenue par PCR est introduite dans le plasmide pINT-MOS en 5' du module d'excision au niveau des sites de restriction *Pac*I et *Asc*I pour donner le plasmide pSUC-5'. Puis, dans ce plasmide pSUC-5' sont introduits alternativement les trois fragments SUC-3'-A, SUC-3'-B et SUC-3'-C en 3' du module d'excision, au niveau des sites de restriction *Fse*I et *Swa*I. Les trois plasmides obtenus pSUC-A (figure 12), pSUC-B et pSUC-C portent respectivement les trois cassettes d'intégration/excision CAS-SUC-A, CAS-SUC-B et CAS-SUC-C.

### B) Délétion de trois allèles du gène SUC par intégrations/excisions multiples

Les trois cassettes CAS-SUC-A, CAS-SUC-B et CAS-SUC-C ont été mises en oeuvre successivement.

### 1) Intégration de la cassette CAS-SUC-A dans le génome de Saccharomyces cerevisiae

La première cassette d'intégration/excision CAS-SUC-A est intégrée dans un locus SUC du génome de *Saccharomyces cerevisiae* par double recombinaison homologue. Pour cela, elle est extraite du plasmide pSUC-A grâce à une digestion par les enzymes *Pac*I et *Swa*I, et purifiée. Une souche de *Saccharomyces cerevisiae* est transformée par l'introduction de la cassette d'intégration/excision sous forme d'ADN linéaire dans les cellules.

Pour sélectionner les cellules où la disruption d'un premier allèle du gène *SUC* a eu lieu, c'est-à-dire les cellules ayant intégré au locus SUC la cassette comprenant le marqueur de sélection kanMX et le module suicide RTA, les transformants sont étalés sur milieu YPD (extrait de levure 1%, bactopeptone 2%, glucose 2%) contenant de la généticine à raison de 200 µg par ml de milieu. Chaque clone ayant intégré la cassette résiste à la généticine et forme colonie. L'intégration spécifique de la cassette CAS-SUC-A au niveau d'un locus SUC conduisant à la délétion d'un premier allèle *SUC* est vérifiée par Southern hybridization (Sambrook *et al*., Molecular Cloning, a Laboratory Manual, Cold Spring Harbor Laboratory Press, 1989, vol.2, pp. 9.31-9.46).

### 2) Excision des séquences hétérologues

Au niveau du locus SUC délété, toutes les séquences hétérologues au génome de *Saccharomyces cerevisiae* doivent être éliminées. L'excision du module d'intégration/excision résulte d'une recombinaison au niveau des 2 séquences SRD et de l'excision de la boucle d'ADN ainsi formée, les souches ayant réalisé cette excision étant sélectionnées grâce à l'induction de l'expression du gène *RTA*. Pour induire l'expression du gène *RTA*, les transformants sont précultivés sur un milieu liquide contenant du galactose comme seule source de carbone puis étalés sur un milieu standard "galactose" gélosé. Ce milieu permet de sélectionner les clones transformants ne produisant pas la toxine codée par le gène *RTA*. Pour contrôler l'élimination du marqueur kanMX, les clones ainsi sélectionnés sont répliqués sur milieu YPD contenant de la généticine (200 µg/ml) pour vérifier la perte de la résistance à la généticine. Cette double sélection permet d'isoler des clones transformants portant une délétion dans un premier locus SUC, ayant excisé les marqueurs de sélection RTA et kanMX.

Après excision du module d'intégration/excision, seule une séquence SRD reste en place, flanquée des séquences SR SUC-5' et SUC-3'-A respectivement en 5' et en 3'. Enfin, la délétion de l'allèle *SUC* et la perte des marqueurs de sélection sont vérifiées par séquençage.

### 3) Intégration et excision des cassettes CAS-SUC-B et CAS-SUC-C

Les deuxième et troisième cassettes d'intégration/disruption CAS-SUC-B et CAS-SUC-C sont utilisées successivement pour déléter une deuxième copie puis une troisième copie génomique du locus SUC, dans la souche de *Saccharomyces cerevisiae* précédemment construite dont une première copie d'un locus SUC a été disruptée. Les cassettes CAS-SUC-B et CAS-SUC-C utilisées successivement pourront s'intégrer au niveau de loci SUC, préférentiellement au niveau d'allèles non transformés, compte tenu du changement de SR en 3' du site d'intégration.

Les cassettes CAS-SUC-B et CAS-SUC-C sont extraites des plasmides pSUC-B et pSUC-C grâce à une digestion par les enzymes *Pac*I et *Swa*I, puis purifiées. Le protocole d'intégration/excision décrit ci-dessus pour CAS-SUC-A est répété successivement pour CAS-SUC-B puis CAS-SUC-C dans des conditions identiques, à savoir transformation et sélection des transformants ayant intégré la cassette, sélection des transformants ayant excisé les marqueurs de sélection. Enfin l'effet des délétions aux loci SUC est contrôlé par dosage de l'activité invertase résiduelle et l'élimination des marqueurs de sélection est vérifiée par séquençage et par hybridation (Southern hybridization).

### EXEMPLE 5 : Délétion des gènes URA3 dans la souche diploïde Y55.LD1.

Une autre application de la cassette est la construction d'un mutant auxotrophique pour l'uracile de la souche de levure Y55.LD1 (souche de laboratoire diploïde, prototrophe, isogénique de la souche Y55 décrite par McCusker, J.H. et J.E. Haber (1988) Cycloheximide-resistant temperature sensitive lethal mutations of *Saccharomyces cerevisae*. Genetics 119 : 303-315). Par rapport à la souche d'origine Y55, la souche Y55.LD1 porte une délétion homozygote du gène *HO*, cette délétion est décrite par Hunter, N., Chambers, S.R., Louis, E. et R.H. Borts (1996) The mismatch repair system contributes to meiotic sterility in an interspecific yeast hybrid. EMBO J. 15 : 1726-1733. Pour transformer cette souche avec des vecteurs levure portant comme marqueur le gène *URA3*, il est nécessaire de rendre cette souche auxotrophe pour l'uracile. Pour cela les deux copies du gène *URA3* présentes dans cette souche sont délétées à l'aide d'une cassette d'intégration/excision selon l'invention.

Le module d'excision selon l'invention est assemblé entre des séquences recombinogéniques capables de se fixer sur le site chromosomique visé par recombinaison avec les séquences homologues présentes dans le gène *URA3* de *S. cerevisiae*. Les deux copies du gène *URA3* présentes dans la souche Y55.LD1 sont délétées successivement avec la même cassette d'intégration/excision appelée CAS-URA.

### A/ Principe de construction de la cassette d'intégration-excision.

De façon analogue à ce qui est décrit ci-dessus pour une cassette d'intégration/excision CAS-SUC, la cassette CAS-URA est composée d'un module d'excision et des deux séquences recombinogéniques SR choisies dans le gène *URA3* de manière à ce que la double recombinaison remplace la région codante du gène par le module d'excision. La première séquence SR située à l'une des extrémités de la cassette d'intégration est un fragment URA3-5' d'env. 600 pb homologue à la région comprenant le promoteur du gène *URA3*. La deuxième séquence SR située à l'extrémité opposée de la cassette d'intégration est un fragment URA3-3' d'env. 600 pb et est homologue à la région du terminateur en 3' de la région codante du gène *URA3*.

La cassette CAS-URA est issue du plasmide pINT-MOS dans lequel les fragments recombinogéniques sont insérés de part et d'autre du module d'excision. Pour ce faire les fragments URA3-5' et URA3-3' sont amplifiés par PCR en utilisant de l'ADN génomique de la souche de laboratoire Y55.LD1. Lors de la PCR, un site de restriction *Pac*I est introduit en 5' du fragment URA3-5' et le site de restriction *Asc*I est introduit en 3' du fragment URA3-5'. De même, le fragment URA3-3' amplifié par PCR est flanqué en 5' d'un site de restriction *Fse*I et en 3' du site de restriction *Swa*I.

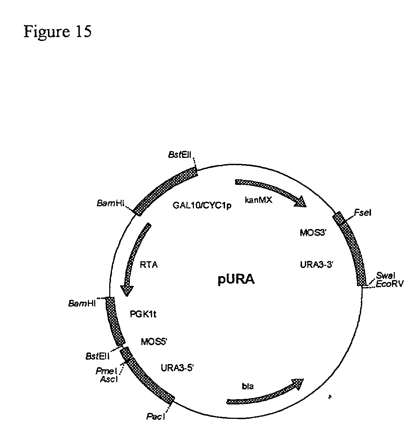

La séquence recombinogénique URA3-5' obtenue par PCR est introduite dans le plasmide pINT-MOS en 5' du module d'excision au niveau des sites de restriction *Pac*I et *Asc*I pour donner le plasmide pURA-5'. Dans ce plasmide pURA-5' est introduit le fragment URA3-3' en 3' du module d'excision, au niveau des sites de restriction *Fse*I et *Swa*I. Le plasmide obtenu est appelé pURA (figure 15).

### B/ Délétion des deux allèles du gène URA3 par intégration/excision multiples

La souche Y55.LD1 est transformée par l'introduction de la cassette d'intégration/excision CAS-URA sous forme d'ADN linéaire. Cette cassette CAS-URA est extraite du plasmide pURA grâce à une digestion par les enzymes de restriction *Pac*I et *Swa*I et purifiée.

L'intégration de la cassette dans un locus d'un gène *URA3*, puis l'excision du module d'intégration/excision sont vérifiées grâce à la présence des 2 marqueurs dominants selon les protocoles décrits dans l'exemple 4.

L'intégration et l'excision de la cassette CAS-URA au niveau du locus du second gène *URA3* sont opérées de la même manière.

L'effet des délétions aux loci URA est contrôlé sur boîte de Pétri contenant un milieu minimal 2 % glucose en vérifiant l'absence de croissance. L'élimination des marqueurs de sélection est vérifiée par séquençage et hybridation (Southern hybridization) sur les cellules haploïdes de plusieurs tétrades obtenues après sporulation de la souche diploïde modifiée.

La souche obtenue est appelée Y55.LD2.

### EXEMPLE 6: Construction et mise en oeuvre de cassettes d'intégration/excision pour l'insertion multiple d'un gène d'intérêt codant pour une protéine non produite par Saccharomyces cerevisiae

On procède comme dans l'exemple 4 ou 5 si ce n'est qu'au site de restriction *Pme*I des plasmides pSUC-A, pSUC-B, pSUC-C ou pURA, on introduit dans la cassette un fragment d'ADN constitué de la partie codante du gène d'intérêt, ou son ADNc, sous promoteur et terminateur de *Saccharomyces cerevisiae*, ledit fragment à introduire dans la cassette d'intégration/excision étant flanqué à chaque extrémité d'un site *Pme*I.

On peut introduire successivement par exemple trois copies de l'ADNc codant pour une enzyme comme la xylanase *Xyl2* ou *Xln2* de *Trichoderma reesei* dans trois loci SUC de *Saccharomyces cerevisiae*, et vérifier d'une part la production de la xylanase par les souches transformées et l'absence de tout ADN exogène à l'exception de celui correspondant à la partie codante du gène *Xyl2* ou *Xln2* de Trichoderma. On peut également choisir des loci URA3 pour l'intégration, étant entendu qu'il est indispensable de garder au moins un locus URA3 actif, si on ne veut pas que la souche devienne auxotrophe pour l'uracile.

### EXEMPLE 7: Construction d'un vecteur d'expression levure sans séquences hétérologues ou exogènes inutiles

Le module composé de deux séquences mosaïques et du marqueur négatif dominant est utilisé pour construire un plasmide navette *E. coli*-levure à taux élevé de copies.

Une fois introduit dans la levure, une recombinaison homologue entre les deux séquences mosaïques conduit à la perte de toutes les séquences issues de *E. coli* ainsi que celles du marqueur négatif.

Le plasmide restant comprend uniquement des séquences de levure et le gène d'intérêt. Il peut être utilisé pour l'expression dudit gène avec un taux élevé de copies en l'absence de tout ADN étranger à la levure si ce n'est celui du gène d'intérêt, si c'est un gène hétérologue.

Le plasmide pINT-MOS (figure 8) sert de base pour la construction de ce plasmide navette. Un oligonucléotide linker (ou séquence de liaison) contenant les sites uniques *Eco*RI, *Asc*I, *Pme*I, *Swa*I, *Pac*I et *Hind*III est introduit entre les deux SRD mosaïques de ce plasmide. (Un site unique est un site présent une seule fois dans une construction).

*Eco*RI et *Hind*III servent respectivement à l'intégration des séquences de l'origine de réplication levure 2 micron et du gène *URA3*. Les sites de restriction *Asc*I, *Pme*I, *Swa*I et *Pac*I donneront des sites potentiels pour l'intégration d'une cassette d'expression. Pour cela, tous ces sites déjà présents dans le plasmide pINT-MOS doivent être éliminés. Pour l'élimination des sites *Eco*RI, *Swa*I et *Eco*RV, le plasmide pINT-MOS est digéré par les enzymes de restriction *Swa*I et *Eco*RV et re-ligué. Plusieurs autres sites de restriction sont remplacés par un site *Bst*EII par digestion du plasmide résultant avec les endonucléases *Pme*I et *Pvu*II, en éliminant avec cette étape le fragment comprenant ces deux sites ainsi que tous les sites de restriction présents entre deux (*Hind*III, *Asc*I, *Sal*I and *Pac*I). Le fragment linéaire est lié à un linker oligonucléotidique conprenant les 7 pb de la séquence de reconnaissance de l'endonucléase *Bst*EII (GGTCACC).

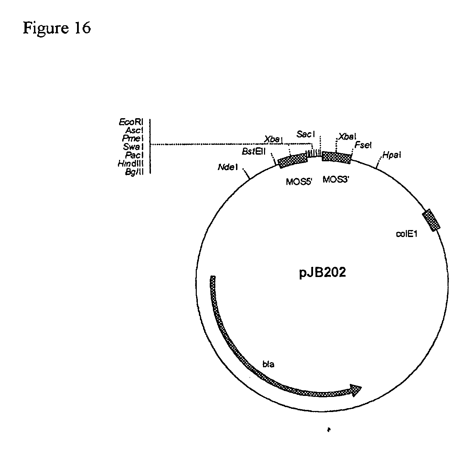

Les marqueurs de séléction positif et négatif sont ensuite remplacés par deux séquences de liaison ou linkers *(SEQ ID No: 3 et 4*) préalablement hybridées entr'elles et apportant les sites de restriction définis ci-dessus. Pour cela, le plasmide est digéré avec les endonucléases *Bgl*II et *Sac*I, le fragment contenant les deux marqueurs est éliminé et le vecteur restant est lié au fragment constitué des deux linkers définis ci-dessus et hybridés (ou liés) entr'eux. Le plasmide résultant est nommé pJB202 (figure 16). Comme indiqué ci-dessus, le fragment obtenu par hybridation des 2 séquences SEQ ID No 3 et 4, introduit dans ce plasmide les sites de restriction *Eco*RI, *Asc*I, *Pme*I, *Swa*I, *Pac*I et *Hind*III ainsi que les sites de restriction *Sac*I et *Bgl*II qui sont reconstitués.

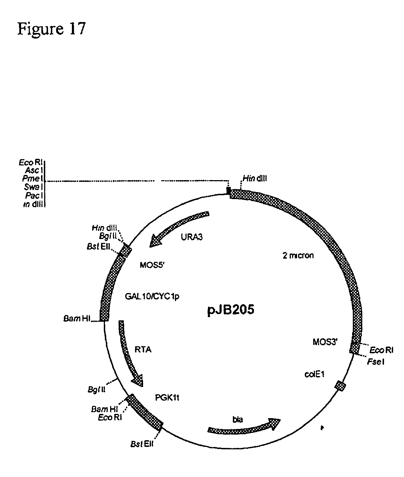

Le gène *URA3* est isolé du plasmide YEp24 (D. Botstein, Falco, S.C., Stewart, S., Brennan, M., Scherer, S., Stinchcomb, D.T., Struhl, K. et R. Davis (1979) Sterile host yeasts (SHY) : a eukaryotic system of biological containment for recombinant DNA experiments. Gene 8 : 17-24) aprés digestion de YEp24 avec *Hind*III. Le fragment résultant, de taille 1,2 kpb, est inséré au niveau du site *Hind*III du plasmide pJB202, donnant le plasmide pJB203. Le fragment 2 micron est également isolé a partir du plasmide YEp24. Pour cela, le plasmide YEp24 est digéré avec *Eco*RI et le fragment de 2.2 kpb contenant l'origine de réplication 2 micron est isolé. Ce fragment est lié au plasmide pJB203, préalablement linéarisé avec *Eco*RI. Ce plasmide pJB204 est digéré par l'endonuclease *Bst*EII et lié avec le produit de PCR décrit dans l'exemple 2 comprenant le module RTA. Le plasmide résultant est nommé pJB205 et est montré en figure 17. Il contient le fragment comprenant l'origine de replication de *E.* coli ainsi que la région codante de la β-lactamase *(bla)* ou *(Amp*^{*r*}*)* du plasmide pSP72 (Promega SA, USA) lié au module RTA, tous les deux séparés par deux séquences SRD mosaiques du module qui ne contient que de l'ADN de la levure.

Le plasmide pJB205 ainsi construit pourra servir à transformer des souches de levures auxotrophes pour l'uracile. On peut concevoir ce vecteur de manière à ce qu'il permette de transformer des souches de levures prototrophes. Dans ce cas, il faut remplacer le gène *URA3* par un marqueur dominant positif d'origine levure, c'est à dire homologue pour la levure. De tels marqueurs sont cités ci-dessus dans la description. Un tel plasmide est appelé ci-après pJB208.

### EXEMPLE 8 : Expression de l'enzyme lactate déhydrogénase dans S. cerevisiae sur plasmide replicatif donnant un nombre élevé de copies

Le gène *ldhL*, codant pour la lactate déshydrogénase, de *Lactobacillus plantarum* est introduit avec les éléments d'expression levure nécessaires dans le plasmide pJB205 décrit dans l'exemple 7.

La cassette d'expression du gène *ldhL* de *L. plantarum* sous contrôle du promoteur et terminateur *ADH1* est obtenu à partir du plasmide pVT.LDHp.

Ce plasmide pVT.LDHp est construit en introduisant dans le plasmide pVT 100-U (T. Vernet, D. Dignard and D.Y. Thomas (1987) A family of yeast expression vectors containing the phage f1 intergenic region. Gene 52 : 225-233), au niveau de son site *Xba*I, un produit de PCR comprenant la région codante du gène *ldhL* de *Lactobacillus plantarum*, amplifié par PCR du plasmide pGIT005 (T. Ferain, D. Garmyn, N. Bernard, P. Hols and J. Delcour (1994) *Lactobacillus plantarum ldhL* Gène : Overexpression and Deletion. J. Bacteriol. 176 : 596-601) et un site *Xba*1 à ses deux extrémités.

La cassette d'expression du gène *ldhL* est amplifiée par PCR à partir du plasmide pVT.LDHp en choisissant 2 amorces introduisant le site *Pme*I aux 2 extrémités du produit de la PCR.

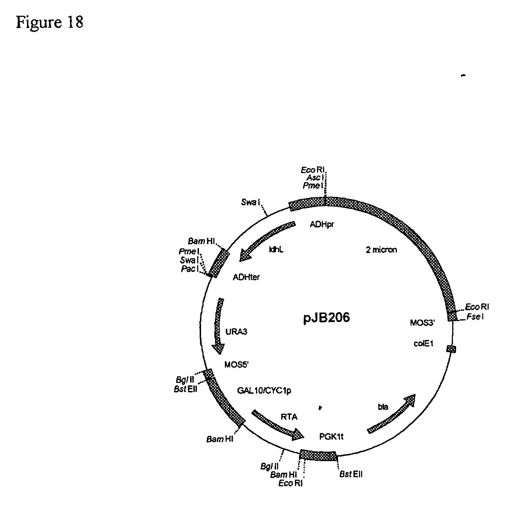

Ce dernier produit de la PCR est purifié, digéré avec l'endonucléase *Pme*I et lié au plasmide pJB205, préalablement linéarisé par digestion avec *Pme*I pour obtenir le plasmide pJB206 (figure 18).

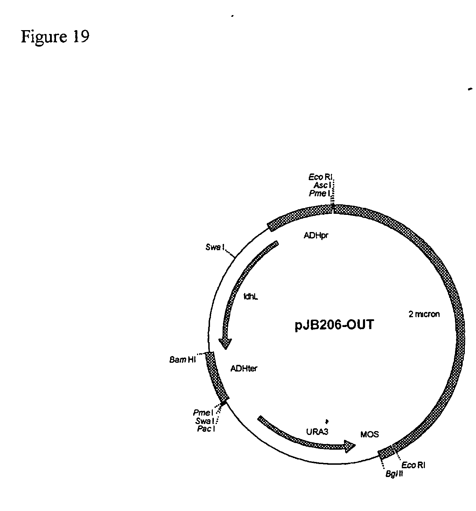

La souche de levure boulangère Y55.LD2 (2n *Δura3::MOS*) construite dans l'exemple 5 est transformée avec le plasmide pJB206. Les transformants sont sélectionnés sur milieu minimal contenant 2% de galactose comme source carbonée. Le galactose présent dans le milieu induit l'expression du marqueur dominant négatif, et permet de sélectionner les cellules portant un ou des plasmides ayant perdu le fragment d'ADN portant ce marqueur négatif et l'ADN d'origine de *E. coli* par recombinaison homologue entre les deux fragments mosaïques. Ce milieu permet de sélectionner des transformants contenant le plasmide pJB206-OUT (figure 19).

Pour la vérification de la perte du marqueur négatif et du fragment d'ADN de *E. coli*, par exicision, le ou les plasmides sont isolés à partir d'un transformant et analysés par PCR et séquençage.

Pour tester la capacité de production d'acide lactique de ce clone, le transformant est cultivé pendant 48 heures sur milieu minimal contenant 5 % glucose comme source carbonée jusqu'à une DO₆₀₀ de 4. Les cellules sont éliminées du milieu par centrifugation et la présence d'acide lactique est dosée par le kit correspondant fourni par Roche Diagnostics (Allemagne). Par rapport à la souche témoin non transformée, le transformant produit des quantités significatives d'acide lactique dans ces conditions.

L'expression du gène codant pour la lactate-dehydrogénase peut également être réalisée en utilisant une souche de levure prototrophe comme la souche de levure Y55.LD1 transformée avec un plasmide navette de type pJB208, où le marqueur de complémentation d'auxotrophie comme le gène *URA3* est remplacé par un marqueur dominant positif levure homologue à la levure. Dans ce cas, l'excision de toutes les séquences d'origine *E. coli*, du marqueur négatif inductible, et d'une copie des 2 séquences SR mosaïques, va résulter de manière similaire en l'obtention d'un plasmide de type pJB209-out comportant uniquement :
- une origine de réplication 2 micron
- un marqueur homologue levure
- le système d'expression du gène d'intérêt
- une séquence mosaïque.

## Revendications

1. Cassette d'ADN destinée à la transformation des levures de manière à ne laisser dans la levure aucun ADN exogène autre que le ou les gènes d'intérêt comportant au moins :
- un marqueur dominant négatif ;
- deux séquences répétées directes (SRD) non exogènes et non recombinogéniques avec le génome de la souche hôte, ces 2 séquences répétées directes étant disposées de part et d'autre du fragment contenant le marqueur dominant négatif;
- éventuellement au moins un gène d'intérêt, avec, si besoin, les éléments nécessaires à son expression dans la cellule hôte.

2. Cassette d'ADN selon la revendication 1 destinée à l'intégration d'au moins un gène d'intérêt ou à l'inactivation d'un gène dans les levures, **caractérisée en ce qu'**elle comporte :
- le marqueur dominant négatif de sélection, ou de préférence deux marqueurs dominants de sélection : un marqueur positif et un marqueur négatif ;
- les deux séquences répétées directes (SRD) non exogènes et non recombinogéniques avec le génome de la souche hôte, ces deux séquences répétées directes flanquant le ou les marqueurs de sélection ;
- deux séquences d'ADN recombinogéniques (SR) correspondant au site d'insertion désiré dans la levure, flanquant en 5' et 3' les deux susdites séquences répétées directes ;
- éventuellement au moins un gène d'intérêt, comportant, si besoin, les éléments nécessaires à son expression dans la cellule hôte, placé entre une séquence SRD et la séquence d'ADN recombinogénique (SR) adjacente.

3. Cassette d'ADN selon la revendication 1 constituée par un plasmide navette E. coli-levure, de préférence E. coli-S. cerevisiae comportant les 2 séquences répétées directes SRD disposées de manière à séparer le plasmide circulaire en 2 régions :
- une région destinée à être éliminée par excision et comportant le marqueur dominant négatif, une origine de réplication d'E. coli, un marqueur de sélection pour E. coli.
- une région destinée à devenir un plasmide multicopie dans la levure comportant une origine de réplication 2 micron, un marqueur de sélection d'origine levure, un système d'expression d'au moins un gène d'intérêt.

4. Cassette d'ADN sous forme d'un plasmide multicopie levure susceptible d'être obtenu après excision du plasmide navette E. coli-levure de la revendication 3 et comportant une séquence SRD comme défini dans la revendication 1, une origine de réplication 2 micron, un marqueur de sélection d'origine levure, un système d'expression d'au moins un gène d'intérêt.

5. Cassette d'ADN selon l'une des revendications 1 à 4 **caractérisée en ce que** le marqueur dominant négatif est un marqueur inductible dont le produit du gène est toxique pour la cellule hôte.

6. Cassette d'ADN selon l'une des revendications 2 et 5 comprenant deux marqueurs dominants de sélection, **caractérisée en ce que** le marqueur de sélection positif est un marqueur de résistance appartenant au groupe des gènes de résistance aux antibiotiques ou aux molécules toxiques pour l'hôte.

7. Cassette d'ADN selon l'une des revendications 3, 4 et 5, **caractérisée en ce que** le marqueur de sélection d'origine levure, est un marqueur de complémentation d'auxotrophie.

8. Cassette selon l'une des revendications 1 à 7, **caractérisée en ce que** la ou les séquences SRD sont des séquences mosaïques constituées par l'assemblage de fragments de diverses séquences d'ADN d'une souche de levure appartenant au même genre et de préférence à la même espèce que la souche hôte.

9. Cassette selon l'une des revendications 1 à 7, **caractérisée en ce que** la ou les séquences SRD sont constituées par une ou des séquences d'ADN non présente(s) dans la souche industrielle, mais présente(s) dans d'autres souches appartenant au même genre, de préférence à la même espèce.

10. Cassette selon la revendication 9, **caractérisée en ce que** la ou les séquences SRD sont constituées par un ou des fragments du gène *MEL1*.

11. Cassette selon la revendication 8, **caractérisée en ce que** chaque fragment des séquences SRD comporte moins de 30 pb.

12. Cassette selon l'une des revendications 1 à 11, **caractérisée en ce que** la ou les séquences SRD comportent de 80 à 300 pb.

13. Cassette selon la revendication 12, **caractérisée en ce que** la ou les séquences SRD comportent au plus environ 200 pb.

14. Cassette selon l'une des revendications 1 à 13, **caractérisée en ce que** le marqueur négatif est un gène suicide inductible *RTA* sous la dépendance d'un promoteur *GAL*, de préférence le promoteur *GAL10*, et encore de préférence le promoteur *GAL10-CYC1*.

15. Cassette selon l'une des revendications 1 à 13, **caractérisée en ce que** le marqueur négatif est un gène inductible codant pour une histone ou toute protéine similaire dont la surexpression est capable de désorganiser une fonction de la cellule et d'inhiber sa croissance.

16. Cassette selon la revendication 15 où le marqueur négatif est le gène H1-1 d'Arabidopsis thaliana placé sous promoteur conditionnel ou inductible.

17. Cassette selon l'une des revendications 1 à 16, **caractérisée en ce que** les séquences SRD sont non codantes.

18. Cassette selon l'une des revendications 2, 5, 6, 8 à 17, **caractérisée en ce qu'**elle ne comporte aucun ADN exogène entre les SRD et les séquences recombinogéniques adjacentes.

19. Cassette selon l'une des revendications 1 à 18, **caractérisée en ce qu'**elle comporte au moins 3 sites de restriction rares et de préférence au moins 5 sites de restriction rares.

20. Procédé d'intégration du gène d'intérêt ou d'inactivation d'un gène dans une levure, **caractérisé en ce que** :
a. on transforme ladite levure à l'aide d'une construction d'ADN consistant en la cassette d'intégration/excision selon l'une des revendications 2, 5, 6, 8 à 19.
b. on sélectionne les levures ayant intégré ladite cassette grâce au marqueur positif.
c. puis, après culture, on sélectionne parmi ces levures les levures dans lesquelles la cassette a été excisée grâce aux séquences SRD en sélectionnant les levures dépourvues du marqueur négatif.

21. Procédé d'intégration de plusieurs copies ou d'inactivation des différentes copies d'un gène dans une levure, **caractérisé en ce qu'**on répète le procédé de la revendication 20 avec des cassettes d'intégration/excision qui comportent pour chaque répétition du procédé des séquences d'ADN recombinogéniques différentes choisies de façon à ce que chaque site comportant une intégration ou étant inactivé ne puisse faire l'objet d'une recombinaison avec la cassette suivante.

22. Procédé de transformation d'une levure auxotrophe avec un plasmide navette selon la revendication 7 afin d'obtenir l'expression d'un nombre élevé de copies d'au moins un gène d'intérêt **caractérisé en ce que** :
a. on transforme la levure avec un plasmide navette selon la revendication 7
b. on sélectionne sur milieu minimal permettant l'induction du marqueur négatif, les cellules ne portant que des plasmides dont la partie correspondant aux fragments E. coli et au marqueur négatif ont été excisées.

23. Levure transformée par une cassette selon l'une des revendications 1 à 19 ou obtenue par le procédé selon l'une des revendications 20 à 22 et qui ne contient que de l'ADN de levure, à l'exception éventuellement du ou des gènes codant pour une protéine d'intérêt, la ou les séquences SRD de la cassette étant non codantes.

24. Levure selon la revendication 23, **caractérisée en ce qu'**au moins un gène a été inactivé par la cassette selon l'une des revendications 1, 2, 5, 6, 8 à 19.

25. Levure selon la revendication 24, **caractérisée en ce que** l'ensemble des copies du même gène ont été inactivées.

26. Levure selon l'une des revendications 23 à 25, **caractérisée en ce qu'**il s'agit de souches de Saccharomyces.

## Patentansprüche

1. DNA-Kassette, die bestimmt ist für die Transformation von Hefen dergestalt, dass in der Hefe keinerlei exogene DNA außer dem oder den Genen von Interesse zurückbleibt, umfassend wenigstens:
- einen dominanten negativen Marker;
- zwei nicht-exogene und mit dem Genom des Wirtsstamms nicht rekombinogene direkte repetitive Sequenzen (SRD), wobei diese 2 direkten repetitiven Sequenzen auf beiden Seiten des Fragments, das den dominanten negativen Marker enthält, angeordnet sind;
- gegebenenfalls wenigstens ein Gen von Interesse mit, sofern erforderlich, den für dessen Expression in der Wirtszelle erforderlichen Elementen.

2. DNA-Kassette nach Anspruch 1, die für die Integration von wenigstens einem Gen von Interesse oder die Inaktivierung eines Gens in Hefen bestimmt ist, **dadurch gekennzeichnet, dass** sie umfasst:
- den dominanten negativen Selektionsmarker oder vorzugsweise zwei dominante Selektionsmarker: einen positiven Marker und einen negativen Marker;
- die zwei nicht-exogenen und mit dem Genom des Wirtsstamms nicht-rekombinogenen direkten repetitiven Sequenzen (SRD), wobei diese zwei direkten repetitiven Sequenzen den oder die Selektionsmarker flankieren;
- zwei rekombinogene DNA-Sequenzen (SR) entsprechend der in der Hefe gewünschten Insertionsstelle, die 5' und 3' die zwei genannten direkten repetitiven Sequenzen flankieren;
- gegebenenfalls wenigstens ein Gen von Interesse, das, sofern erforderlich, die für dessen Expression in der Wirtszelle erforderlichen Elemente umfasst, angeordnet zwischen einer SRD-Sequenz und der angrenzenden rekombinogenen DNA-Sequenz (SR).

3. DNA-Kassette nach Anspruch 1, gebildet aus einem E. coli-Hefe-, vorzugsweise E. coli-S. cerevisiae-Shuttle-Plasmid, das die zwei direkten repetitiven SRD-Sequenzen dergestalt angeordnet aufweist, dass das zirkuläre Plasmid in 2 Regionen geteilt wird:
- eine Region, die dazu bestimmt ist, durch Herausschneiden (Exzision) eliminiert zu werden und die den dominanten negativen Marker, einen Replikationsstartpunkt aus E. coli, einen Selektionsmarker für E. coli umfasst;
- eine Region, die dazu bestimmt ist, ein Plasmid mit hoher Kopienzahl in der Hefe zu werden, umfassend einen 2 Mikron-Replikationsstartpunkt, einen Selektionsmarker von Hefe-Ursprung, ein Expressionssystem von wenigstens einem Gen von Interesse.

4. DNA-Kassette in Form eines Hefe-Plasmids mit hoher Kopienzahl, das durch Herausschneiden (Exzision) aus dem E. coli-Hefe-Shuttle-Plasmid von Anspruch 3 erhalten werden kann und das eine SRD-Sequenz wie in Anspruch 1 definiert, einen 2 Mikron-Replikationsstartpunkt, einen Selektionsmarker von Hefe-Ursprung, ein Expressionssystem von wenigstens einem Gen von Interesse umfasst.

5. DNA-Kassette nach einem der Ansprüche 1 bis 4, **dadurch gekennzeichnet, dass** der dominante negative Marker ein induzierbarer Marker ist, dessen Genprodukt für die Wirtszelle toxisch ist.

6. DNA-Kassette nach einem der Ansprüche 2 und 5, umfassend zwei dominante Selektionsmarker, **dadurch gekennzeichnet, dass** der positive Selektionsmarker ein Resistenzmarker ist, der aus der Gruppe der Resistenzgene gegen Antibiotika oder gegen für den Wirt toxische Moleküle stammt.

7. DNA-Kassette nach einem der Ansprüche 3, 4 und 5, **dadurch gekennzeichnet, dass** der Selektionsmarker von Hefe-Ursprung ein Auxotrophie-Komplementationsmarker ist.

8. Kassette nach einem der Ansprüche 1 bis 7, **dadurch gekennzeichnet, dass** die SRD-Sequenz oder -Sequenzen Mosaiksequenzen sind, die durch das Zusammenfügen von Fragmenten von unterschiedlichen DNA-Sequenzen von einem Hefestamm, der aus derselben Gattung und vorzugsweise derselben Spezies wie der Wirtsstamm stammt, gebildet werden.

9. Kassette nach einem der Ansprüche 1 bis 7, **dadurch gekennzeichnet, dass** die SRD-Sequenz oder -Sequenzen durch eine oder mehrere DNA-Sequenzen gebildet werden, die in dem industriellen Stamm nicht vorhanden ist bzw. sind, aber in anderen Stämmen, die aus derselben Gattung, vorzugsweise derselben Spezies stammen, vorhanden ist bzw. sind.

10. Kassette nach Anspruch 9, **dadurch gekennzeichnet, dass** die SRD-Sequenz oder -Sequenzen durch ein oder mehrere Fragmente des *MEL1*-Gens gebildet werden.

11. Kassette nach Anspruch 8, **dadurch gekennzeichnet, dass** jedes Fragment der SRD-Sequenzen weniger als 30 bp umfasst.

12. Kassette nach einem der Ansprüche 1 bis 11, **dadurch gekennzeichnet, dass** die SRD-Sequenz oder -Sequenzen 80 bis 300 bp umfassen.

13. Kassette nach Anspruch 12, **dadurch gekennzeichnet, dass** die SRD-Sequenz oder -Sequenzen höchstens etwa 200 bp umfassen.

14. Kassette nach einem der Ansprüche 1 bis 13, **dadurch gekennzeichnet, dass** der negative Marker ein induzierbares *RTA*-Suizidgen unter der Abhängigkeit von einem GAL-Promotor, vorzugsweise dem *GAL10*-Promotor und ferner bevorzugt dem *GAL10-CYC1*-Promotor ist.

15. Kassette nach einem der Ansprüche 1 bis 13, **dadurch gekennzeichnet, dass** der negative Marker ein induzierbares Gen ist, das ein Histon oder ein jegliches ähnliches Protein kodiert, dessen Überexpression in der Lage ist, eine Funktion der Zelle zu stören bzw. zu zerstören und deren Vermehrung zu hemmen.

16. Kassette nach Anspruch 15, worin der negative Marker das unter einen konditionalen oder induzierbaren Promotor gestellte H1-1-Gen von Arabidopsis thaliana ist.

17. Kassette nach einem der Ansprüche 1 bis 16, **dadurch gekennzeichnet, dass** die SRD-Sequenzen nicht-kodierend sind.

18. Kassette nach einem der Ansprüche 2, 5, 6, 8 bis 17, **dadurch gekennzeichnet, dass** sie keinerlei exogene DNA zwischen den SRD und den angrenzenden rekombinogenen Sequenzen umfasst.

19. Kassette nach einem der Ansprüche 1 bis 18, **dadurch gekennzeichnet, dass** sie wenigstens 3 seltene Restriktionsstellen und vorzugsweise wenigstens 5 seltene Restriktionsstellen umfasst.

20. Verfahren zur Integration des Gens von Interesse oder zur Inaktivierung eines Gens in eine(r) Hefe, **dadurch gekennzeichnet, dass**:
a. man die Hefe mit Hilfe eines DNA-Konstrukts, bestehend aus der Integrations/Exzisions-Kassette gemäß einem der Ansprüche 2, 5, 6, 8 bis 19, transformiert.
b. man die Hefen, die die Kassette integriert haben, dank des positiven Markers selektiert.
c. man dann nach einer Kultivierung unter diesen Hefen die Hefen selektiert, in denen die Kassette dank der SRD-Sequenzen herausgeschnitten worden ist, indem man die Hefen, denen der negative Marker fehlt, selektiert.

21. Verfahren zur Integration einer Mehrzahl von Kopien oder zur Inaktivierung der verschiedenen Kopien eines Gens in eine(r) Hefe, **dadurch gekennzeichnet, dass** man das Verfahren des Anspruchs 20 wiederholt mit Integrations/Exzisions-Kassetten, die für jede Wiederholung des Verfahrens unterschiedliche rekombinogene DNA-Sequenzen umfassen, die dergestalt ausgewählt sind, **dass** jede Stelle, die eine Integration aufweist oder inaktiviert ist, nicht den Gegenstand einer Rekombination mit der folgenden Kassette bilden kann.

22. Verfahren zur Transformation einer auxotrophen Hefe mit einem Shuttle-Plasmid nach Anspruch 7, um die Expression einer erhöhten Anzahl von Kopien von wenigstens einem Gen von Interesse zu erzielen, **dadurch gekennzeichnet, dass**:
a. man die Hefe mit einem Shuttle-Plasmid nach Anspruch 7 transformiert
b. man auf Minimalmedium, das die Induktion des negativen Markers erlaubt, die Zellen selektiert, die lediglich Plasmide tragen, deren den E. coli-Fragmenten und dem negativen Marker entsprechender Teil herausgeschnitten worden ist.

23. Hefe, die durch eine Kassette nach einem der Ansprüche 1 bis 19 transformiert oder durch das Verfahren nach einem der Ansprüche 20 bis 22 erhalten worden ist und die lediglich Hefe-DNA enthält mit Ausnahme von gegebenenfalls dem oder den ein Protein von Interesse kodierenden Genen, wobei die SRD-Sequenz oder -Sequenzen der Kassette nicht-kodierend ist bzw. sind.

24. Hefe nach Anspruch 23, **dadurch gekennzeichnet, dass** wenigstens ein Gen durch die Kassette nach einem der Ansprüche 1, 2, 5, 6, 8 bis 19 inaktiviert worden ist.

25. Hefe nach Anspruch 24, **dadurch gekennzeichnet, dass** die Gesamtheit der Kopien dieses Gens inaktiviert worden ist.

26. Hefe nach einem der Ansprüche 23 bis 25, **dadurch gekennzeichnet, dass** es sich um Saccharomyces-Stämme handelt.

## Claims

1. DNA cassette intended for the transformation of yeasts so as to leave in the yeast no exogenous DNA other than the gene(s) of interest containing at least:
- one negative dominant marker;
- two direct repeat sequences (DRS) which are nonexogenous and nonrecombinogenic with the genome of the host strain, these 2 direct repeat sequences being placed on either side of the fragment containing the negative dominant marker;
- optionally at least one gene of interest, with, if necessary, the elements necessary for its expression in the host cell.

2. DNA cassette according to Claim 1, intended for the integration of at least one gene of interest or for the inactivation of a gene in yeasts, **characterized in that** it contains:
- the negative dominant selectable marker, or preferably two dominant selectable markers: one positive marker and one negative marker;
- the two direct repeat sequences (DRS) which are nonexogenous and nonrecombinogenic with the genome of the host strain, these two direct repeat sequences flanking the selectable marker(s);
- two recombinogenic DNA sequences (RS) corresponding to the desired insertion site in the yeast, flanking in 5' and 3' the abovementioned two direct repeat sequences;
- optionally at least one gene of interest, containing, if necessary, the elements necessary for its expression in the host cell, placed between a DRS sequence and the adjacent recombinogenic DNA sequence (RS).

3. DNA cassette according to Claim 1, consisting of an E. coli-yeast, preferably E. coli-S. cerevisiae, shuttle plasmid containing the 2 direct repeat sequences (DRS) placed so as to separate the circular plasmid into 2 regions:
- a region intended to be removed by excision and containing the negative dominant marker, an E. coli replication origin, a selectable marker for E. coli;
- a region intended to become a multicopy plasmid in the yeast containing a 2 micron replication origin, a selectable marker of yeast origin, a system for expressing at least one gene of interest.

4. DNA cassette in the form of a yeast multicopy plasmid which can be obtained after excision of the E. coli-yeast shuttle plasmid of Claim 3 and containing a DRS sequence as defined in Claim 1, a 2 micron replication origin, a selectable marker of yeast origin, a system for expressing at least one gene of interest.

5. DNA cassette according to one of Claims 1 to 4, **characterized in that** the negative dominant marker is an inducible marker whose gene product is toxic for the host cell.

6. DNA cassette according to either of Claims 2 and 5 comprising two dominant selectable markers, **characterized in that** the positive selectable marker is a resistance marker belonging to the group of genes for resistance to antibiotics and to molecules which are toxic for the host.

7. DNA cassette according to one of Claims 3, 4 and 5, **characterized in that** the selectable marker of yeast origin is a marker for complementation of auxotrophy.

8. Cassette according to one of Claims 1 to 7, **characterized in that** the DRS sequence(s) are mosaic sequences consisting of the assembly of fragments of various DNA sequences of a yeast strain belonging to the same genus and preferably to the same species as the host strain.

9. Cassette according to one of Claims 1 to 7, **characterized in that** the DRS sequence(s) consist of one or more DNA sequences not present in the industrial strain, but present in other strains belonging to the same genus, preferably to the same species.

10. Cassette according to Claim 9, **characterized in that** the DRS sequence(s) consist of one or more fragments of the *MEL1* gene.

11. Cassette according to Claim 8, **characterized in that** each fragment of the DRS sequences contains less than 30 bp.

12. Cassette according to one of Claims 1 to 11, **characterized in that** the DRS sequence(s) contain from 80 to 300 bp.

13. Cassette according to Claim 12, **characterized in that** the DRS sequence(s) contain at most about 200 bp.

14. Cassette according to one of Claims 1 to 13, **characterized in that** the negative marker is an *RTA* inducible suicide gene under the control of a *GAL* promoter, preferably the *GAL10* promoter, and more preferably the *GAL10-CYC1* promoter.

15. Cassette according to one of Claims 1 to 13, **characterized in that** the negative marker is an inducible gene encoding a histone or any similar protein whose overexpression is capable of disorganizing a function of the cell and of inhibiting its growth.

16. Cassette according to Claim 15, where the negative marker is the Arabidopsis thaliana H1-1 gene placed under a conditional or inducible promoter.

17. Cassette according to one of Claims 1 to 16, **characterized in that** the DRS sequences are noncoding.

18. Cassette according to one of Claims 2, 5, 6, 8 to 17, **characterized in that** it contains no exogenous DNA between the DRSs and the adjacent recombinogenic sequences.

19. Cassette according to one of Claims 1 to 18, **characterized in that** it contains at least 3 rare restriction sites and preferably at least 5 rare restriction sites.

20. Method of integrating a gene of interest or of inactivating a gene in a yeast, **characterized in that**:
a. the said yeast is transformed with the aid of a DNA construct consisting of the integration/excision cassette according to one of Claims 2, 5, 6, 8 to 19,
b. the yeasts having integrated the said cassette are selected by means of a positive marker,
c. and then, after culturing, the yeasts in which the cassette has been excised by virtue of the DRS sequences are selected among these yeasts by selecting the yeasts lacking the negative marker.

21. Method of integrating several copies or of inactivating different copies of a gene in a yeast, **characterized in that** the method of Claim 20 is repeated with integration/excision cassettes which contain, for each repetition of the method, different recombinogenic DNA sequences chosen such **that** each site containing an integration or being inactivated cannot be the subject of a recombination with the next cassette.

22. Method for transforming an auxotrophic yeast with a shuttle plasmid according to Claim 7 in order to obtain the expression of a large number of copies of at least one gene of interest, **characterized in that**:
a. the yeast is transformed with a shuttle plasmid according to Claim 7,
b. the cells carrying only the plasmids from which the part corresponding to the E. coli fragments and to the negative marker have been excised are selected on minimal medium allowing the induction of the negative marker.

23. Yeast transformed with a cassette according to one of Claims 1 to 19 or obtained by the method according to one of Claims 20 to 22 and which contains only yeast DNA, with the possible exception of the gene(s) encoding a protein of interest, the DRS sequence(s) of the cassette being noncoding.

24. Yeast according to Claim 23, **characterized in that** at least one gene was inactivated by the cassette according to one of Claims 1, 2, 5, 6, 8 to 19.

25. Yeast according to Claim 24, **characterized in that** all the copies of the same gene were inactivated.

26. Yeast according to one of Claims 23 to 25, **characterized in that** it is a Saccharomyces strain.
